(19) **Europäisches Patentamt / European Patent Office / Office européen des brevets**

(11) **EP 1 327 442 B1**

(12) **EUROPEAN PATENT SPECIFICATION**

(45) Date of publication and mention
of the grant of the patent:
**16.12.2009 Bulletin 2009/51**

(51) Int Cl.:
***A61K 8/02*** *(2006.01)*     ***A61Q 19/00*** *(2006.01)*

(21) Application number: **00966525.8**

(22) Date of filing: **16.10.2000**

(86) International application number:
**PCT/JP2000/007179**

(87) International publication number:
**WO 2002/032405 (25.04.2002 Gazette 2002/17)**

(54) **PROCESS FOR PRODUCING GEL SHEET FOR APPLICATION TO LIVING BODY, GEL SHEET FOR APPLICATION TO LIVING BODY OBTAINED BY THE PRODUCTION PROCESS, AND METHOD OF SKIN CARE WITH THE SAME**

PROZESS ZUR HERSTELLUNG EINES GELBLATTES ZUR ANWENDUNG AM LEBENDEN KÖRPER, GELBLATT ZUR ANWENDUNG AM LEBENDEN KÖRPER ERHALTEN DURCH DIESEN HERSTELLUNGSPROZESS UND METHODE ZUR HAUTPFLEGE MIT DIESEM BLATT.

TECHNIQUE DE PRODUCTION DE FEUILLE DE GEL POUR APPLICATION A UN CORPS VIVANT, FEUILLE DE GEL POUR APPLICATION A UN CORPS VIVANT OBTENUE PAR CETTE TECHNIQUE DE PRODUCTION, ET PROCEDE DE SOINS DE LA PEAU A L'AIDE DE CETTE FEUILLE DE GEL

(84) Designated Contracting States:
**FR GB**

(43) Date of publication of application:
**16.07.2003 Bulletin 2003/29**

(73) Proprietors:
- **SEKISUI KASEIHIN KOGYO KABUSHIKI KAISHA**
  **Osaka-shi,**
  **Osaka 530-0047 (JP)**
- **Kanebo Cosmetics, Inc.**
  **Tokyo 108-8080 (JP)**

(72) Inventors:
- **HIGASHI, Takashi**
  **Sakurai-shi, Nara 633-0091 (JP)**
- **HAYASHI, Yasushi**
  **Hachioji-shi, Tokyo 192-0904 (JP)**
- **KURODA, Akihiro**
  **Odawara-shi, Kanagawa 250-0865 (JP)**
- **MAEDA, Saori**
  **Kawasaki-shi, Kanagawa 215-0021 (JP)**

(74) Representative: **Modiano, Micaela Nadia**
**Modiano Josif Pisanty & Staub Ltd**
**Thierschstrasse 11**
**80538 München (DE)**

(56) References cited:
| | |
|---|---|
| WO-A-95/30411 | JP-A- 4 178 323 |
| JP-A- 60 226 808 | JP-A- 2000 119 129 |
| JP-A- 2000 226 325 | |

- **DATABASE WPI Section Ch, Week 199413 Derwent Publications Ltd., London, GB; Class A96, AN 1994-106740 XP002385289 & JP 06 056660 A (NIPPON SURFACTANT KOGYO KK) 1 March 1994 (1994-03-01)**
- **DATABASE WPI Section Ch, Week 199732 Derwent Publications Ltd., London, GB; Class B02, AN 1997-347392 XP002385301 & JP 09 143070 A (NIPPON FLOUR MILLS CO LTD) 3 June 1997 (1997-06-03)**
- **DATABASE WPI Section Ch, Week 198947 Derwent Publications Ltd., London, GB; Class A96, AN 1989-343216 XP002385291 & JP 01 254612 A (SUZUKI NIHONDO KK) 11 October 1989 (1989-10-11)**
- **DATABASE WPI Section Ch, Week 199121 Derwent Publications Ltd., London, GB; Class A96, AN 1991-152277 XP002385292 & JP 03 086806 A (NIPPON OILS & FATS CO LTD) 11 April 1991 (1991-04-11)**

**Description**

Technical field

The present invention relates to a process for producing adhesive gel sheet for application to a living body, gel sheet for application to a living body obtained by the production process. The adhesive gel sheet for a living body according to the present invention can suitably be used in the fields of drugs, quasi drugs, cosmetics, hygienic goods and sundries.

Further, the present invention relates to a skin-care method using the above-described adhesive gel sheet for a living body, wherein various medicinal ingredients (skin-care ingredients) having ameliorating effects on a variety of skin troubles, such as roughness, dryness, somberness, rings under the eyes and freckles, are effectively worked in a simple manner and in a short time.

Background Art

So far, various kinds of medicinal ingredients having ameliorating effects on various troubles on a skin surface have been developed.

Such medicinal ingredients have been used by containing them in a skin lotion, impregnating them in a base material such as nonwoven or woven fabric or blending them into a peel-off type pack agent.

However, the former two processes have a problem in that the medicinal ingredients do not sufficiently permeate into the skin. Further, since the peel-off type pack agent is used in such a manner that a film formed by drying is peeled off, a sufficient amount of solvent such as water cannot be retained in the film, which lacks a moisturizing feeling and a cooling feeling during application. Moreover, user's fingers may be soiled upon application. The pack agent needs to be applied uniformly so that it is peeled off cleanly without remains, which requires experience, and thus a problem in handling also arises.

Japanese Unexamined Patent Publication No. SHO 54-49334 describes a pack agent which can be peeled off without drying after the application. This pack agent can retain liquid in a greater amount. However, there are problems in that the fingers are soiled upon application and the pack agent, which is soft and not solidified enough, is not easy to peel off cleanly.

To solve the above-described problems, there has been proposed in the field of poultices an application form (a gel sheet) in which ointment in a gel state containing the medicinal ingredients is coated on a base material such as nonwoven fabric, from the viewpoint of excellent adhesive and occlusive properties to the skin and ensured liquid retaining ability. In recent years, a gel sheet similar to the poultices has been widely used for skin-care applications.

For example, Japanese Unexamined Patent Publication No. SHO 55-92306 proposes a pack agent in the form of a gel sheet containing a skin-beautifying ingredient and a tackifying ingredient in a water-containing synthetic polymer sheet. Further, Japanese Unexamined Patent Publication No. HEI 1-254612 proposes a pack agent in the form of a gel sheet made of polyacrylic acid or salts thereof, water, a skin-beautifying ingredient, an aluminum salt and polyalcohol.

A crosslinking reaction of polyacrylic acid or salts thereof with polyvalent metal ions such as an aluminum salt, which occurs particularly in the latter gel sheet, is immediately started by mixing them. Therefore, heating is not necessary, which is advantageous in that even the medicinal ingredients lacking stability can be blended. However, in order to obtain a homogeneous gel sheet by mixing both of them homogeneously in a common stirrer, the stirring needs to be continued for such a long time as 15 minutes or longer in a liquid state. Therefore, the rate of reaction between the polyacrylic acid and the polyvalent metal salt needs to be reduced. As a result, a considerably long time is required for solidification, which causes a problem of deterioration in production efficiency.

Further, as described above, the prior art gel sheet is a laminate of the gel and the support base of the nonwoven or woven fabric, as typified by the poultice. As the support base, an opaque material having little opening area ratio is used to prevent strike-through of the gel. Accordingly, there is a problem in that the gel sheet causes a visually uncomfortable feeling to a large extent upon application to the skin and hence time and place for application are limited to a large degree. In the case of a sheet made of nonwoven or woven fabric impregnated with a skin lotion, the nonwoven or woven fabric needs to have a greater Base weight per m$^2$ (Metsuke weight) in order to retain liquid in a greater amount. This makes the resulting sheet opaque, which causes the visually uncomfortable feeling upon application to the skin.

Further, in the case of the gel sheet as used in the poultice in which polyacrylic acid or salts thereof is crosslinked using polyvalent metal ions such as an aluminum salt, both substances are selected so that the reaction occurs at a low reaction rate. Therefore, the gel itself is apt to be emulsified and rendered opaque after the crosslinking.

In this connection, Japanese Unexamined Patent Publication No. 2000-119129 proposes a cosmetic gel sheet in which a support base made by laminating a transparent film and pulp or rayon is stacked with a gel layer. The gel sheet has a structure in which the transparent film takes charge of preventing the strike-through of the gel and sustaining the strength as the support base, while the pulp or rayon as a fiber component takes charge of bonding with the gel.

Since this sheet includes the film, even pulp or rayon having a greater opening area ratio can be used and hence the transparency is ensured to a certain degree. However, the transparency is still insufficient and the film is poor in elasticity, which is problematic in that the sheet is hard to fit sufficiently with an elaborately curved plane such as a face.

In this connection, Japanese Unexamined Patent Publication No. HEI 3-86806 proposes a pack agent in the form of a multilayer gel sheet using a gel made of gelatin (support base) and a gel made of polyacrylic acid or the like. In this case, a soft gel is used as the support base and the transparency thereof is ensured, thereby reducing the visually uncomfortable feeling upon application to a certain degree. However, in order to prevent the gel layers which are hard to bond from coming unstuck, the two gel layers need to be solidified simultaneously. This is problematic because the production steps and facilities are complicated. Further, the gel used as the support base can easily be finished in transparent, whereas the polyacrylic acid gel involves the above-described problems in finishing in transparent. Therefore, as a whole, the gel sheet is still insufficient in transparency.

Thus, the adhesive gel sheets for a living body which have been used in the prior art skin-care processes are not totally satisfactory in moisture retaining ability, cooling feeling, adhesion property and occlusive feeling to the skin, reduction of the visually uncomfortable feeling, as well as ease of production.

Disclosure of Invention

Thus, according to the present invention, there is provided a process for producing an adhesive gel sheet for a living body, comprising mixing a synthetic polymer compound that dissolves in an amount of 1g or more in 100 g of water at 20°C, having an anionic functional group, with a polycationic compound capable of crosslinking the polymer compound in the presence of an aqueous medium to prepare a gel-forming composite having a pH of 3 to 7, impregnating the gel-forming composite into a woven or nonwoven fabric while the viscosity of the composite is 1,000 to 30,000 mPa·s, shaping the composite-impregnated fabric into a sheet and crosslinking the composite in this state to obtain an adhesive gel sheet for a living body having a transparent or translucent polymer gel, the crosslinking thereof being completed in this state to obtain a polymergel solidified in one piece with the woven or nonwoven fabric, subjecting the adhesive gel sheet to curing treatment using a crosslinking agent on an optional part of at least one of the surfaces of the adhesive gel sheet wherein the woven or nonwoven fabric has a hypothetical shielding area N ($m^2/m^2$) by fibers per $m^2$ in the range of $0.05 \leq N \leq 2.0$ wherein N is represented by the following equation:

$$N = 0.107W \sqrt{\frac{1}{d\rho}}$$

wherein W is a Base weight per $m^2$ of the woven or nonwoven fabric represented by $g/m^2$, d is a denier amount of fibers comprising the fabric an $\rho$ is a density of a component of the fibers represented by $g/cm^3$, and $3 \leq W \leq 50$ $g/m^2$.

According to the above-described process, an adhesive gel sheet for a living body having excellent characteristics can be provided by a simple producing process.

Further, according to the present invention, there is provided an adhesive gel sheet for a living body obtained by the above-described process, wherein the medicinal ingredient exists at least on a surface to be adhered to a living body. The adhesive gel sheet for a living body according to the present invention is characterized in that

(1) it totally satisfies moisture retaining ability, cooling feeling, adhesion property and occlusive feeling to the skin,
(2) it stays on the skin surface without peeling or falling off even if an excessive adhesion ability is not given thereto and causes the action of the medicinal ingredient to the skin more effectively,
(3) it is easily applied and has transparency to such a degree that a visually uncomfortable feeling is not caused, and
(4) it exudes a solution contained in the gel sheet to the gel surface upon application to the skin, in particular between the skin and the gel sheet, as moisture on a gel surface opposite to the skin is volatized and contraction in volume occurs.

Brief Description of Drawings

Fig. 1 is a schematic view illustrating a structure of an adhesive gel sheet for a living body according to the present invention;

Fig. 2 is a schematic view illustrating a structure of the adhesive gel sheet for a living body according to the present invention;

Fig. 3 is a schematic view illustrating a structure of the adhesive gel sheet for a living body according to the present invention;

Fig. 4 is a schematic view illustrating a structure of the adhesive gel sheet for a living body according to the present invention;

Fig. 5 is a schematic view illustrating a structure of a producing apparatus used for producing the adhesive gel sheet for a living body according to the present invention;

Fig. 6 is a schematic view illustrating a structure of the producing apparatus used for producing the adhesive gel sheet for a living body according to the present invention; and

Fig. 7 is a schematic enlarged view illustrating part of the producing apparatus used for producing the adhesive gel sheet for a living body according to the present invention.

Mode for Carrying Out the Invention

As a result of analysis on time points of forming into the sheet, the present invention provides a process for producing a sheet, wherein a gel-forming composite in part of which a polymer gel is formed is shaped into a sheet when the gel-forming composite has a viscosity of such a degree that allows the gel-forming composite to be easily spread onto a base (a support base or a mold) for maintaining the gel-forming composite in the sheet form, and then the shape retainability thereof is maintained. Since the gel-forming composite has a viscosity in the above-described range, an adhesive gel sheet for a living body having excellent properties can be obtained by a simple process.

The adhesive gel sheet for a living body obtained by the process of the present invention is comprised of a polymer gel (hereinafter merely referred to as a gel) which is obtained by mixing a water soluble synthetic polymer compound having an anionic functional group and a polycationic compound capable of crosslinking the polymer compound in the presence of an aqueous medium to prepare a gel-forming composite, shaping the gel-forming composite into a sheet on a support base or in a mold while the viscosity thereof is 1,000 to 50,000 mPa·s and completing the crosslinking thereof in this state.

If the viscosity is higher than 50,000 mPa·s, there arises difficulty in shaping the gel-forming composite freely into the sheet form or the like and hence it is not preferred. If the viscosity is lower than 1,000 mPa·s, the gel-forming composite drips from the support base or is easily transformed by external force and hence it is not preferred.

According to the present invention, "an adhesive gel sheet for a living body" signifies an adhesive sheet or a sticky sheet which is applied onto a skin for the purposes of cosmetic and facial treatment and skin treatment, such as a pack agent, an adhesive patch, a transdermal absorption agent, an adhesive tape for a living body, a wound dressing and an antiphlogistic analgesic agent. Hereinafter, it may simply be referred to as a gel sheet.

The viscosity is measured using a B-type viscometer (BH type, manufactured by TOKIMEC INC.) at liquid temperature of 25°C and the number of revolutions of 4 rpm.

[Polymer gel]

The polymer gel is characterized in that it is a colorless or colored and transparent or translucent substance and has a shape retainability.

Upon application to a skin surface, the polymer gel preferably exudes liquid contained in the gel to the surface thereof.

As the gel comprising the adhesive gel sheet for a living body, natural polymer gel bodies such as carragheenan, gelatin, sodium alginate and pectin are usable. However, since the natural polymer gel bodies are poor in thermal stability and easily become rotten, synthetic polymer gel bodies are more preferable.

Here, "transparent or translucent" signifies a state in which black characters of 10 points written on a white background can be read through the gel sheet placed on the characters.

[Synthetic polymer compound comprising the gel]

The water soluble synthetic polymer compound having an anionic functional group (hereinafter it may be merely referred to as a polymer compound) is not particularly limited as long as it has an affinity for water and reacts with a polycationic compound to form a network structure while retaining at least water to be able to form a gel, and as long as it has been used in the fields of cosmetics, drugs, quasi drugs, hygienic goods and sundries for transdermal application. Various kinds of polymer compounds can be used. The anionic functional group is preferably at least one selected from a carboxyl group and a sulfone group. Here, the water solubility signifies that the compound dissolves in an amount of 1 g or more in 100 g of water at 20°C.

Above all, from the viewpoint of easy production, suitably used is a water soluble synthetic polymer compound which has at least one kind of functional group selected from the carboxyl group and the sulfone group on a side chain and is obtained by polymerization of one or not less than two kinds of unsaturated polymerizing monomers. In this case, unsaturated polymerizing monomers other than those having the carboxyl group and the sulfone group on the side chain may be copolymerized to such a degree that the resulting polymer compound does not lose the water solubility.

Examples of the above-described unsaturated polymerizing monomers having a carboxyl group on the side chain include (meta)acrylic acid and that in which the carboxyl group is partially or completely neutralized with alkali such as sodium hydroxide. Examples of the unsaturated polymerizing monomers having a sulfone group on the side chain include t-butyl acrylamide sulfonic acid and that in which the sulfone group thereof is partially or completely neutralized with alkali such as sodium hydroxide.

Other than the carboxyl group and the sulfone group, the above-described water soluble synthetic polymer compound may have a functional group obtained by modifying them. For example, in order to improve adhesion to the skin, about 40 % of the carboxyl group in the water soluble synthetic polymer compound may be esterified to have an affinity for oil. Further, in the case where a long saturated hydrocarbon portion exists on the side chain, a carboxyl group, a sulfonic acid group, a hydroxyl group, an amido group or an amino group may be introduced or treatment with reagents such as acid or alkali may be performed so as to give an affinity for water to the portion. Such procedures may be carried out before or after the polymerization of the monomers.

The crosslinking factor for introducing a network structure to the water soluble synthetic polymer compound to form a polymer gel is a polycationic compound. Suitably used is a polyvalent metal ion compound having a valence of 3 or more.

As the polyvalent metal ion compound, for example, aluminum compounds, calcium compounds and magnesium compounds may be considered. Among them, in view of providing an optimum reaction rate for the crosslinking efficiency and the gel formation, suitable are compounds which are hardly soluble to water and contain trivalent aluminum ions, such as aluminum hydroxide and salts thereof, synthetic aluminum silicate, magnesium aluminometasilicate, magnesium aluminum oxides, aluminum oxides, dihydroxyaluminum aminoacetate, magnesium aluminum hydroxide carbonate hydrate and aluminum hydroxide-sodium carbonate coprecipitate. These compounds having an amorphous structure are more preferable. The term hardly soluble signifies that the compound is hardly dissolved in normal water, but can be dissolved in an amount of 0.1 g or more in 100 g of acidic water having a pH of 3.5 or lower at 20°C.

The blending amount of the polymer compound for comprising the polymer gel is preferably 0.5 to 50 % by mass with respect to the total amount of the polymer gel. If the blending amount of the polymer compound is below 0.5 % by mass, strength of the gel decreases and the shape retainability of the gel becomes unstable. Accordingly, the resulting adhesive gel sheet for a living body may possibly be torn upon use thereof, which deteriorates the ease of use. On the other hand, if the amount exceeds 50 % by mass, the gel strength improves but the polymer structure becomes too dense, thereby the liquid amount retained in the gel may be reduced too much. Among the above-described range, in particular, the blending amount of the polymer compound is preferably 1 to 30 % by mass, more preferably 2 to 25 % by mass.

In the case of the polymer gel which exudes a solution containing the medicinal ingredient to the gel surface upon application to the skin surface, the blending amount of the polymer compound is preferably smaller, such as 0.5 to 15 % by mass.

The blending amount of the polycationic compound is preferably 0.1 to 10 % by mass with respect to the polymer gel. If the blending amount of the polycationic compound is below the above-described range, the strength of the gel decreases and the shape retainability of the gel becomes unstable. Accordingly, the resulting adhesive gel sheet for a living body may possibly be torn upon use thereof, which deteriorates the ease of use. On the other hand, if the blending amount exceeds the above-described range, the gel becomes more fragile and the gel may easily be torn or broken by tensile stress or compressive stress.

[Solvent comprising the gel]

The polymer gel contains at least water as a solvent. The ratio of water in the polymer gel is preferably 1 to 99.5 % by mass. If the ratio of water in the gel falls short of 1 % by mass, various additives to be blended in the gel such as medicinal ingredients may not be easily dissolved. On the other hand, if the water ratio in the gel is higher than 99.5 % by mass, the strength of the gel decreases and the shape retainability of the gel becomes unstable. Accordingly, the resulting adhesive gel sheet for a living body may possibly be torn upon use thereof, which deteriorates the ease of use. Among the above-described range, in particular, the ratio of water in the gel is preferably 5 to 95 % by mass, more preferably 10 to 85 % by mass.

As the solvent comprising the polymer gel, other solvents than water may be used after being mixed with water as long as they do not induce phase separation in the gel-forming composite and have been used in the fields of cosmetics, drugs, quasi drugs, hygienic goods and sundries for transdermal application. As such solvents, considered are, for example, monoalcohols such as ethyl alcohol, grycols such as 1,3-butylene grycol, polyalcohols such as glycerin, polyglycols in which two or more molecules of glycols and polyalcohols are linked through ether linkage and polyglycerins. These may be used solely or in combination of two or more kinds.

The ratio of the other solvent than water in the polymer gel is preferably 98 % by mass or lower. If the ratio exceeds 98 % by mass, the content of water as the solvent is reduced and various additives blended in the gel may not be easily dissolved. Further, the strength of the gel decreases and the shape retainability of the gel becomes unstable. Accordingly, the resulting adhesive gel sheet for a living body may be torn upon use thereof, which deteriorates the ease of use.

In the case of the polymer gel which exudes a solution containing the medicinal ingredient to the gel surface upon application to the skin surface, the blending amount of the solvent is preferably larger, such that the total amount of water and the solvent other than water is 85 to 99.5 % by mass. In this case, usable solvents other than water may be ethers of polyalcohols and polyalcohols. Preferable examples thereof are diethylene glycol, triethylene glycol, polyethylene glycol having average molecular weight of 6,000 or lower, propylene glycol, dipropylene glycol, tripropylene grycol, polypropylene glycol having average molecular weight of 1,000 or lower, 1,3-butylene glycol, hexylene glycol, isoprene glycol, polyoxypropylene diglycelylether and polyoxypropylene glycelylether having average addition mole of 9 or lower.

[Crosslinking reaction]

The polymer gel is obtained by mixing a polycationic compound into a solvent containing at least water and the polymer compound having the anionic functional group to prepare a gel-forming composite and crosslinking the gel-forming composite.

Here, the pH upon mixing is in the range of 3 to 7. If the pH upon mixing is lower than the above-described range, the pH of the resulting gel sheet after the crosslinking becomes too low, which adversely affects the skin. Further, there is also a problem in that the crosslinking reaction rate becomes too high and the gel is solidified while the polycationic compound and the polymer compound are not yet mixed homogeneously. If the pH upon mixing is higher than the above-described range, the crosslinking reaction rate is low, which deteriorates the production efficiency. In addition, there is a problem in that the polycationic compound which is hardly soluble to water is apt to remain in the gel in a whitish state, which renders the resulting sheet opaque. More preferable pH range upon mixing is 3.5 to 6.0.

The crosslinking reaction is preferably performed using a mixing apparatus which is capable of stirring the polymer compound and the polycationic compound continuously.

Making use of the low reaction rate, it is also possible to mix the polymer compound and the polycationic compound in advance at a pH higher than the range of 3 to 7, and then react both compounds in the mixing apparatus capable of continuous stirring at a pH adjusted within the range of 3 to 7 by adding acid homogeneously. In this case, any acid may be used as long as it does not cause serious harmful effect on the skin and is added in such an amount that finally adjusts the pH in the range of 3 to 7. Usable are various kinds of inorganic and organic acids such as hydrochloric acid, acetic acid, citric acid and malic acid. The mixing of the polymer compound and the polycationic compound, which is not followed by a remarkable curing reaction, can be carried out using a normal batch type stirring apparatus.

According to the present invention, the polymer gel is characterized by being transparent or translucent. However, as described above, the polycationic compound whose solubility to water is very low is apt to remain in the gel in a whitish state even after the gel is solidified. On the other hand, if the polycationic compound having high solubility to water is used or the gel-forming composite having a low pH is prepared, a highly transparent gel is obtained. However, the crosslinking reaction of the gel-forming composite is apt to proceed rapidly. Accordingly, if the mixing is carried out in a vessel using the batch type stirring apparatus, the gel may possibly be solidified to such a degree that it cannot be molded after the stirring.

According to the gel sheet of the present invention, the mixing of the polymer compound and the polycationic compound in the presence of an aqueous medium, which proceeds in a short reaction time, can be carried out in a short time using a mixing apparatus capable of continuous stirring. For instance, the reaction time is preferably adjusted so that an increase in viscosity accompanying the crosslinking reaction reaches to 50,000 mPa·s or higher in 10 minutes after the mixing of the gel-forming composite is started. Further, the mixing apparatus capable of continuous stirring signifies a stirring apparatus in which the introduction of the object to be mixed, the mixing thereof and the taking-out of the resulting mixture are continuously performed. More specifically, a static mixer and a reactive extruder are signified. Among them, the static mixer is preferable because reduction in molecular weight due to shearing of the polymer hardly occurs.

Stirring time in the apparatus, i.e., retention time in the apparatus, is desirably about 5 to 300 seconds, more desirably about 10 to 150 seconds. If the retention time is shorter than the above-described range, the mixing is apt to be performed non-homogeneously. Further, if the retention time is longer than the above-described range, a system of the polymer compound and the polycationic compound, in which the crosslinking reaction rate is high, causes the gel to be solidified easily in the apparatus. Further, the size of the apparatus is enlarged as the retaining amount increases.

[Medicinal ingredient]

A liquid existing at least on a skin-adhering surface of the adhesive gel sheet for a living body according to the present invention is at least comprised of a medicinal ingredient and a solvent (hereinafter referred to as a medicinal ingredient solution). The medicinal ingredient is not particularly limited as long as it has been used, for example, in the fields of drugs, quasi drugs, cosmetics, hygienic goods and sundries for the purposes of cosmetic and facial treatment and skin treatment.

For example, considered are angelica keiskei extract, persea gratissima (avocado) fruit extract, hydrangea serrata leaf extract, althea extract, arnica extract, aloe extract, prunus armeniaca (apricot) kernel extract, prunus armeniaca (apricot) kernel extract, ginkgo biloba extract, foeniculum vulgare (fennel) extract, turmeric extract, oolong tea extract, rose fruit extract, echinacea angustifolia leaf exract, scutellaria baicalensis root extract, phellodendron bark extract, coptis japonica root extract, hordeum vulgare seed extract, hypericum perforatum extract, lamium album extract, nasturtium officinale extract, citrus aurantium dulcis (orange) fruit extract, dehydrated seawater, seaweed extract, hydrolyzed elastin, hydrolyzed wheat powder, hydrolyzed silk, chamomilla recutita (matricaria) extract, daucus carota sativa (carrot) root extract, artemisia capillaris flower extract, glycyrrhiza extract, hibiscus tea extract, pyracantha fortuneana fruit extract, actinidia chinensis (kiwi) fruit extact, cinchona extract, cucumis sativus (cucumber) fruit extract, guanosine, gardenia florida extract, sasa veitchii extract, sophora angustifolia extract, walnut shell extract, citrus grandis (grapefruit) fruit extract, clematis vitalba leaf extract, chlorella vulgaris extaract, morus alba leaf extract, gentiana lutea extract, black tea extract, yeast extract, arctium lappa root extract, fermented rice bran extract, rice germ oil, symphytum officinale leaf extract, collagen, cowberry extract, asiasarum root extract, family of bupleurum extract, umbilical cord extract, saponaria officinalis extract, bumboo extract, crataegus cuneata fruit extract, zanthoxylum fruit extract, corthellus shiitake (mushroom) extract, rehmania root extract, lithospermum erythrorhizone root extract, perilla herb extract, linden extract, spiraea ulmaria extract, peony root extract, acorus calamus root extract, birch extract, equisetum arvense extract, hedera helix (ivy) extract, quickthorn extract, bourtree extract, nosebleed extract, mentha piperita (peppermint) leaf extract, salvia officinalis (sage) leaf extract, malva sylvestris (mallow) extract, cnidium officinale root extract, swertia japonica extract, glycine soja (soybean) seed extract, jujube fruit extract, thymus vulgaris (thyme) extract, green tea extract, eugenia caryophyllus (clove) flower extract, Imperata cylindrical extract, citrus unshiu peel extract, japanese angelica root extract, calendula officinalis flower extract, peach kernel extract, bitter orange peel extract, houttuynia cordata extract, solanum lycopersicum (tomato) extract, fermented soybeans extract, panax ginseng root extract, allium sativum (garlic) extract, wild rose extract, hibiscus extract, ophiopogonis tuber extract, carum petroselinum (parsley) extract, honey, hamamelis virginiana (witch hazel) extract, parietaria officinalis extract, isodonis japonicus extract, bisabolol, eriobotrya japonica leaf extract, coltsfoot extract, butterbur sprout extract, poria cocos extract, ruscus aculeatus root extract, vitis vinifera (grape) fruit extract, propolis, luffa cylindrica fruit extract, carthamus tinctorius (safflower) flower extract, peppermint extract, tilia platyphyllos flower extract, paeonia suffruticosa root extract, humulus lupulus (hops) extract, pinus sylvestris cone extract, aesculus hippocastanum (horse chestnut) extract, lysichiton camtschatcense extract, sapindus mukurossi peel extract, melissa officinalis (balm mint) leaf extract, prunus persica (peach) leaf extract, centaurea cyanus flower extract, eucalyptus leaf extract, saxifraga sarmentosa extract, yuzu extract, mugwort extract, lavandula angustifolia (lavender) extract, pyrus malus (aaple) fruit extract, lactuca scariola sativa (lettuce) extract, citrus medica limonum (lemon) extract, astragalus sinicus extract, rosa centifolia flower extract, rosamarinus officinalis (rosemary) leaf extract, anthemis nobilis flower extract, royal jelly extract and the like.

Also included are biopolymers such as deoxyribonucleic acid, mucopolysaccharide, sodium hyaluronate, chondroitin sulfate sodium, collagen, elastin, chitin, chitosan and hydrolyzed egg shell membrane; mosturizing ingredients such as amino acid, sodium lactate, urea, sodium pyrrolidone carboxylate, betaine, whey and trimethyl glycine; oil ingredients such as sphingolipid, ceramide, cholesterol, cholesterol derivatives and phospholipid; anti-inflammatory agents such as $\varepsilon$-aminocaproic acid, glycyrrhizinic acid, $\beta$-glycyrrhetinic acid, lysozyme chloride, guaiazulene and hydrocortisone; vitamins such as vitamins A, B2, B6, C, D, E and K, calcium pantothenate, biotin, nicotinamide and vitamin C esters; active ingredients such as allantoin, diisopropylamine dichloroacetate and 4-aminomethyl cyclohexanecarboxylic acid; antioxidants such as tocopherol, carotenoid, flavonoid, tannin, lignan and saponin; cell activators such as $\alpha$-hydroxy acid and $\beta$-hydroxy acid; blood circulation accelerators such as $\gamma$-orizanol and vitamin E derivatives; wound healing agents such as retinol and retinol derivatives; whitening agents such as arbutin, kojic acid, placenta protein, sulfur, ellagic acid, linolic acid, tranexamic acid and glutathione; cepharanthin, glycyrrhiza extract, capsicum tincture, hinokitiol, iodinated garlic extract, pyridoxine hydrochloride, dl-$\alpha$-tocopherol, dl-$\alpha$-tocopherol acetate, nicotinic acid, nicotinic acid derivatives, D-pantothenyl alcohol, acetyl pantothenyl ethyl ether, biotin, allantoin, isopropyl methyl phenol, estradiol, ethinyl estradiol, capronium chloride, benzalkonium chloride, diphenhydramine hydrochloride, takanal,

7

camphor, salicylic acid, vanillylamide nonylate, vanillylamide nonanoate, piroctone olamine, glyceryl pentadecanoate, l-menthol, mononitro guaiacol, resorcin, γ -aminobutyric acid, benzethonium chloride, mexiletine hydrochloride, auxin, estrogen, cantharis tincture, ciclosporin, hydrocortisone, polyoxyethylene sorbitan monostearate, mentha oil, analgesics, tranquilizers, antihypertensive agents, antibiotics, antihistamic agents, antibacterial substances, plant-derived ingredients and seaweed-derived ingredients.

The medicinal ingredient solution may contain other additives than those described above as required, such as humectants, thickeners, flavoring agents, coloring agents, stabilizers, antioxidants, ultraviolet absorbers, tackifiers, pH adjusters, chelating agents, surfactants, antiseptic agents and antibacterial agents.

The humectants include, for example, ethylene glycol, triethylene glycol, propylene glycol, butylene glycol, diethylene glycol, dipropylene glycol, glycerin, diglycerin, triglycerin, ethylene carbonate, sorbitol, maltitol, trehalose, raffinose, xylitol, glucose, fructose, mannitol, hyaluronic acid and salts thereof, glycols such as polyethylene glycol and polyglycerin, polyalcohols and sugar alcohols. These may be used solely or in combination of two or more kinds.

As the thickeners, preferable are those which give a certain degree of viscosity to a mixture solution in a relatively small amount. For example, water soluble polymers such as polyethylene oxide and hydroxylethyl cellulose may be used.

As a solvent comprising the medicinal ingredient solution, a composition which allows homogeneous mixing of the above-described medicinal ingredients and additives is selected from among water which serves as a solvent comprising the above-described polymer gel and solvents which have been used in the fields of cosmetics, drugs, quasi drugs, hygienic goods and sundries for transdermal application. These may be used solely or in combination. Even if the medicinal ingredient or the like does not dissolve in the solvent, it is acceptable as long as the medicinal ingredient is kept dispersed homogeneously in the solvent by the action of a surfactant or a thickener.

The blending amount of the medicinal ingredient is not defined specifically because the optimum use amount varies depending on the kind thereof. In general, the amount is preferably 0.001 to 80 % by mass with respect to the total amount of the medicinal ingredient solution, more preferably 0.05 to 60 % by mass.

The optimum amount of the medicinal ingredient solution cannot be defined specifically because it varies depending on the kind and the amount of the medicinal ingredient contained therein. However, the amount is preferably 0.5 to 50 mg per $cm^2$ of the surface area of the gel surface to be applied to the skin, more preferably 1 to 20 mg because homogeneous effect is caused to the skin and problems in handling such as dripping of liquid upon application of the gel hardly occur. If the liquid amount is too small, the medicinal ingredient is not spread almost over the to-be-applied surface of the gel sheet, which renders the effect of the medicinal ingredient insufficient. Further, if the liquid amount is too large, liquid drips upon application of the gel sheet, which causes a discomfort feeling and wastes the medicinal ingredient.

To quantify the liquid on the gel surface, a paper wiper (Kimwipe wiper S-200 manufactured by CRECIA Corporation) is weighed sheet by sheet using a precision balance, the whole gel surface to be measured is wiped with it and the used wiper is weighed again using the precision balance. A difference between the two weight values is defined as the liquid weight on the gel sheet surface.

It is acceptable that a certain amount of the medicinal ingredient solution exists on the gel upon application of the gel sheet to the skin surface. For example, in the case of supplying the solution to the gel surface from outside by immersing the gel in the solution in a larger amount than the above-described range before application to the skin, it is sufficient as long as the liquid amount within the above-described range remains on the gel surface upon application.

The medicinal ingredient solution may be supplied by coating, dropping or spraying onto the gel after solidification of the gel is completed. Alternatively, the medicinal ingredient solution may be supplied before completing the solidification of the gel, e.g., upon spreading into the sheet form, by coating, dropping or spraying onto one of the sheet surfaces. In the case of adding the medicinal ingredient solution after spreading the gel into the sheet form, the medicinal ingredient solution may be supplied by directly coating, dropping or spraying on the gel surface. If the gel surface is covered with a protection film or the like, the gel surface may be moistened with the medicinal ingredient solution after peeling off the protection film immediately before use. If the gel is packaged as being carried on a base film or a cup larger than itself, the medicinal ingredient solution may be coated or dropped on a board or cup surrounding the gel so that the gel surface can be moistened with the medicinal ingredient solution upon detaching the gel from the base film or the cup for use.

In addition to the above-described process of supplying the medicinal ingredient solution separately from the gel, it is also possible to blend the medicinal ingredient and a solvent capable of dissolving it in advance in the gel-forming composite before solidifying the gel, so that the medicinal ingredient solution can be exuded to the surface through curing treatment after the solidification of the gel.

Further, if the sheet is formed using a mold, the medicinal ingredient solution may be supplied by coating or dropping into the mold in advance.

[Curing treatment]

One of the surfaces of the gel sheet made of the polymer gel may be entirely or partially subjected to curing treatment. As processes for the curing treatment, mentioned are a process of applying a curing agent capable of reacting with the gel to increase the crosslinking density of the gel and a process of irradiating the gel with ultraviolet rays or electron beam to increase the crosslinking density on one of the surfaces of the polymer gel.

Among these processes, the process of applying the curing agent entirely or partially on at least one of the surfaces of the gel sheet (more specifically, the curing agent is coated or applied) is preferable in view of ease of operation and producing cost.

(Curing agent)

As the curing agent, used is that containing a crosslinking factor capable of reacting with the polymer comprising the adhesive gel sheet for a living body and increasing the crosslinking density of the polymer. That is, the curing agent increases the crosslinking density at least entirely or partially on one of the surfaces of the gel sheet made of the polymer gel, thereby exuding the medicinal ingredient solution, i.e., the solvent comprising the gel and the medicinal ingredient dissolved therein, from the inside of the gel. There is no particular limitation to the curing agent as long as it has been used in the fields of cosmetics, drugs, quasi drugs, hygienic goods and sundries for transdermal application.

The curing agent has the function of increasing the crosslinking density of the gel by reacting the crosslinking factor contained in the curing agent with the polymer comprising the polymer gel. Accordingly, in the above-described gel, a polymer compound capable of causing a crosslinking reaction with the crosslinking factor contained in the curing agent needs to exist. As such polymer compound, may be considered a polymer compound having a network structure capable of forming the polymer gel by itself and a polymer compound other than the polymer compound comprising the polymer gel, which is not crosslinked and contained in the gel together with the solvent.

A Combination of the above-described curing agent and the polymer gel is not particularly limited, but in view of ease of production, suitable is a combination wherein:

(i) the curing agent has at least one kind of crosslinking factor selected from the group consisting of polycationic compounds, polycarboxylic acids, polyalcohols, multifunctional epoxides and dialdehydes; and
(ii) the polymer compound comprising the gel is a water soluble synthetic polymer compound obtained by polymerizing one or not less than two kinds of unsaturated polymerizing monomers having at least one kind of functional group selected from the group consisting of carboxyl groups, sulfonic acid groups, hydroxyl groups and amido groups on the side chain (that is, a polymer compound capable of causing a crosslinking reaction with the crosslinking factor of (i)).

Among the polymer compounds comprising the gel as described in (ii), examples of the polymer compounds having a carboxyl group or a sulfonic acid group on the side chain include those described in the above-described section [polymer compound comprising the gel]. Examples of the polymer compounds having a hydroxyl group on the side chain include polyvinyl alcohols obtained by hydrolyzing a polymer of vinyl acetate monomer. Examples of the polymer compounds having an amido group on the side chain include poly (meta)acrylamide, poly N,N'-dimethyl(meta)acrylamide, polyvinylpyrrolidone and poly N-vinylacetamide.

Monomers comprising the above-described polymer compounds may have a functional group modified from a carboxyl group and the like as described in the section [polymer compound comprising the gel]. Treatment for introducing the functional group and the timing of the treatment are the same as described in the above-mentioned section [polymer compound comprising the gel].

The polymer compound as described in (ii) to be reacted with the curing agent may be a polymer compound having a network structure for forming the polymer gel by itself, or may be a polymer compound other than the polymer compound comprising the polymer gel, which is not crosslinked and contained in the gel together with the solvent. To include the non-crosslinked polymer compound in the gel, it is preferred to mix the polymer compound for forming the polymer gel and the polymer compound of (ii) in advance to prepare a polymer solution, and then introduce the network structure to the former.

Among the curing agents described in (i), the polycationic compounds indicate all the compounds containing a cation having a valence of two or more. Among them, in view of efficiency in crosslinking reaction, compounds containing a cation having a valence of three of more, for example, $Al^{3+}$, $Fe^{3+}$, $Ti^{3+}$, $In^{3+}$, $Zr^{4+}$ and $Ta^{5+}$, are suitably used.

The above-described polycationic compounds may be water soluble salts such as aluminum chloride or those hardly soluble to water such as aluminum hydroxide. An optimum one is suitably selected depending on the producing process. In other words, if quick curing is intended, a salt having solubility to the curing agent used or the solvent contained in the polymer gel may be selected. On the other hand, in order to perform the curing treatment for an hour or longer, for example, a salt which is hardly soluble to the curing agent used or the solvent contained in the polymer gel may be selected.

Among the curing agents described in (i), the polycarboxylic acids include, for example, succinic acid, fumaric acid, phthalic acid, citric acid and malic acid. The polyacohols include, for example, ethylene glycol, propylene glycol, butanediol, glycerin, diethylene glycol and diglycerin. The multifunctional epoxides include, for example, ethylene glycol diglycidyl ether, polyethylene glycol diglycidyl ether, propylene glycol diglycidyl ether, polyprolylene glycol diglycidyl ether, polytetramethylene glycol diglycidyl ether, glycerol polyglycidyl ether, polyglycerol polyglycidyl ether, sorbitol polyglycidyl ether, sorbitan polyglycidyl ether, trimethylolpropane polyglycidyl ether, pentaerythritol polyglycidyl ether, resorcin diglycidyl ether, neopentyl glycol diglycidyl ether and 1,6-hexanediol diglycidyl ether. Further, the dialdehydes include, for example, glyoxal, terephthalaldehyde and glutaraldehyde.

With respect to a combination of the polymer compound and the crosslinking factor in the above-described curing treatment, a combination of a polymer compound obtained by polymerizing unsaturated polymerizing monomers having at least an anionic functional group and a crosslinking factor containing at least a polycationic compound is particularly preferable among those described above in view of ease of production. The anionic functional group signifies all the functional groups which are capable of having chemical bond with a cation in water. In view of ease of production, preferable one is a carboxyl group represented by -COOH, -COOX (X; counter ion).

The curing agent to be applied to at least one of the surfaces of the polymer gel comprising the adhesive gel sheet for a living body of the present invention may contain a solvent together with the above-described crosslinking factor. Such a solvent may include, for example, water, monoalcohols such as ethyl alcohol, glycols such as 1,3-butylene glycol, polyalcohols such as glycerin. As described above, the crosslinking factor may be dissolved in the solvent or take the form of slurry without being dissolved. Further, as required, the curing agent may contain various additives such as thickeners, flavoring agents, coloring agents, stabilizers, antioxidants, ultraviolet absorbers, tackifiers, pH adjusters, chelating agents, surfactants, antiseptic agents and antibacterial agents.

The curing treatment according to the present invention is performed to at least one of the surfaces of the gel sheet comprised of the polymer gel. If the sheet is used for application to skin, the treated surface may be a surface contacting the skin or an inverse surface which does not contact the skin. With respect to the to-be-treated surface, the treatment may be performed uniformly on the entire surface or ununiformly in part of the surface. Alternatively, the treatment may be performed only a part of the surface. The curing treatment may be carried out by any processes as long as the crosslinking density in the vicinity of the treated part of the gel surface is increased as compared with the inside of the gel to exude the solution from the inside of the gel. If only a part of the gel surface is subjected to the curing treatment, it is preferable to treat the surface by 10 % or higher of the surface area. If the treated surface area is less than 10%, the solvent exudation from the inside of the gel in response to the curing treatment does not occur remarkably, which possibly makes insufficient the effects of the medicinal ingredients, moisture retainability and a cooling feeling given to the skin.

In the curing treatment, the amount of the curing agent to be applied on the surface on the gel sheet made of the polymer gel is preferably $1 \times 10^{-10}$ to $1 \times 10^{-2}$ equivalent per cm$^2$ of the surface to be treated, as represented by a crosslinking equivalent of the crosslinking factor. If the amount is smaller than $1 \times 10^{-10}$ equivalent/cm$^2$, the solvent exudation from the inside of the gel does not occur remarkably, which possibly makes moisture retainability and a cooling feeling given to the skin insufficient. On the other hand, if the amount reaches to $1 \times 10^{-2}$ equivalent/cm$^2$, the curing treatment is sufficiently performed and does not proceed effectively any more. The crosslinking equivalent of the crosslinking factor mentioned herein signifies the amount of the crosslinking factor at the crosslinking point, which is represented by the number of moles.

In order to exude the solvent containing the medicinal ingredient more effectively from the inside of the polymer gel through the curing treatment of the present invention, the amount (mole number) of the polycationic compound comprising the network structure of the polymer in the gel is preferably 0.001 to 8.0 moles at the crosslinking point, more preferably 0.01 to 5.0 moles with respect to 100, which is the mole number of the monomers comprising the polymer. If the amount of the polycationic compound at the crosslinking point exceeds the above-described range, the original crosslinking density in the gel becomes too high, which impairs the effect of exuding the solvent from the inside of the gel even if the curing treatment is performed. On the other hand, if the amount of the curing agent at the crosslinking point is below the above-described range, the shape retainability of the gel becomes unstable. Accordingly, the resulting adhesive gel sheet for a living body may be torn upon use thereof, which deteriorates the ease of use.

Further, in order to exude the solvent more effectively from the inside of the gel through the curing treatment, the gel preferably contains, as a solvent other than water comprising the polymer gel, a polarized solvent such as glycols such as 1,3-butylene glycol and polyalcohols such as glycerin by 1 % by mass, more preferably by 5 % by mass. By containing the polarized solvent as the solvent for the gel in addition to water, water itself as the solvent for the gel can be exuded easily.

If the matrix of the network structure comprising the gel is too large, the ability of the gel to hold the solvent therein becomes too great and the exudation of the solution from the inside of the gel is attenuated even if the curing treatment is carried out. Therefore, it is preferred that not less than 50 % by mass of the water soluble polymer compound having an anionic functional group, which is involved in the network structure for forming the gel, is comprised of molecules having a molecular weight of 100,000 or smaller. However, if the molecular weight of the water soluble polymer compound is smaller than 1,000 in not less than 50 % by mass thereof, the shape retainability of the gel becomes unstable. Accordingly, the resulting adhesive gel sheet for a living body may be torn upon use thereof, which deteriorates the ease of use.

With respect to the adhesive gel sheet for a living body according to the present invention, the polymer gel capable of exuding the solution containing the medicinal ingredient to the gel surface upon application to the skin surface signifies the one which exudes the solution containing the medicinal ingredient to the gel sheet surface, in particular between the gel sheet and the skin, as the gel sheet contracts due to transpiration (drying) of the solvent comprising the gel upon application to the skin. Therefore, the same effect can be obtained even if the solution containing the medicinal ingredient is not added or the curing treatment is not performed to the surface. It is not necessary that the solution containing the medicinal ingredient exists on the gel sheet surface upon application to the skin as long as the solution exudes to the gel surface during the application. As compared with the above-described adhesive gel sheet for a living body, a moisturizing feeling during application tends to lack. However, this is highly advantageous for production because the addition of the medicinal ingredient solution and the curing treatment are unnecessary. Either type may be selected in consideration of the producing costs and the difference in feeling upon use.

The gel which can be used as the adhesive gel sheet for a living body capable of exuding the solution containing the medicinal ingredient upon application to the skin surface is a gel which satisfies the following conditions. That is, it is a polymer gel which contains a solvent at least containing water, a water soluble synthetic polymer compound and a medicinal ingredient. When this gel is applied on a glass plate at one surface to be kept stand under the condition of 36°C and relative humidity of 60 % for 20 minutes while the other surface is exposed to an atmosphere and then removed from the glass plate, it leaves 0.5 to 50 mg of the medicinal ingredient solution on the glass plate per $cm^2$ of the gel area. The appearance thereof is preferably transparent or translucent.

In the gel satisfying these conditions, polymers and solvents described in the above-described sections [Synthetic polymer compound comprising the gel] and [Solvent comprising the gel] are preferably used. The medicinal ingredients mentioned in the above-described section [Medicinal ingredient] are also preferably used.

[Structure of the gel sheet]

The adhesive gel sheet for a living body according to the present invention is comprised of the polymer gel which contains at least water as a solvent and has a network structure and the solution containing the medicinal ingredient to be present on the surface to be applied to the skin. Other than those described above, a base material such as woven fabric or nonwoven fabric having a sufficiently large open area ratio may be included in the polymer gel for the purpose of improving tear strength and handleability of the gel sheet without impairing the transparency thereof.

To include the woven or nonwoven fabric in the gel, the woven or nonwoven fabric is preferably immersed in a gel-forming composite having a viscosity of 1,000 to 30,000 mPa·s, which is shaped into the sheet and then solidified. More specifically, upon the shaping of the gel-forming composite into the sheet by sandwiching it in a gap between two firm-shaped separators arranged at a predetermined interval, the woven or nonwoven fabric having a sufficiently large open area ratio is preferably immersed in the gel-forming composite in a direction parallel to the separators and the gel and the woven or nonwoven fabric are integrally solidified by crosslinking reaction. Thereby, the woven or nonwoven fabric is included in the gel. By immersing the woven or nonwoven fabric having a large open area ratio in the mixture solution which is not solidified yet, residual air bubbles between fibers are reduced and the woven or nonwoven fabric and the gel are integrated firmly after the solidification.

The state of being "included" in the gel signifies that at least part of the woven or nonwoven fabric is buried in the gel. The woven or nonwoven fabric may completely be buried in the gel, or part of it may be exposed out of the gel.

The film-shaped separator is a component which is generally used as a releasing material such as a plastic film made of polyethylene, polyprolylene or polyethylene terephthalate, a composite of them formed by lamination, a composite thereof with paper and a film on whose surface silicon or the like is applied to give a releasing property. Selection is made among them depending on the releasing property with respect to the gel-forming composite and whether or not the separator is used as a protection film of the gel sheet after the shaping.

The woven or nonwoven fabric having a sufficiently large open area ratio according to the present invention has a Base weight per $m^2$ W in the range of $3 \leqq W \leqq 50$ as represented by $g/m^2$, more preferably in the range of $5 \leqq W \leqq 30$. If the Base weight per $m^2$ is smaller than the range, the strength of the woven or nonwoven fabric is small and it cannot effectively serve as the base material. Further, if the Base weight per $m^2$ is larger than the range, the solidity of the gel increases, which causes problems in that the elasticity of the gel is impaired and the transparency of the gel is lost.

The woven or nonwoven fabric having a sufficiently large open area ratio according to the present invention has a hypothetical shielding area N ($m^2/m^2$) by fibers per $m^2$ in the range of $0.05 \leqq N \leqq 2.0$, which is represented by the following equation:

$$N = 0.107W \sqrt{\frac{1}{d\rho}}$$

(wherein W is a Base weight per $m^2$ of the woven or nonwoven fabric represented by $g/m^2$, d is a denier amount of fibers comprising the fabric and p is a density of a component of the fibers represented by $g/cm^3$). More preferably, the range is $0.1 \leqq N \leqq 1.0$. If the N value is smaller than the above-described range, the grain of the woven or nonwoven fabric becomes too large, which makes difficult to use the fabric as the base material. If the N value is larger than the above-described range, the transparency becomes insufficient and hence it is not preferable.

As the fibers comprising the woven or nonwoven fabric having a large open area ratio, those generally used can be used. However, in view of transparency and hygienic control, synthetic fibers are rather preferable than natural fibers. Among them, preferable are nylon and polyester fibers. Further, if multifilament fibers are used, the number of fibers comprising a single filament is preferably smaller in view of preventing the generation of bubbles upon burying the fabric in the gel. More preferably, monofilament fibers are used.

The thickness of the gel sheet may suitably be adjusted depending on the use conditions thereof. The thickness may be entirely uniform or partially varied. In view of a discomfort feeling upon application to the skin and the strength, the thickness is preferably in the range of 0.1 to 3 mm. If the thickness of the entire gel sheet is lower than 0.1 mm, the strength of the gel may possibly be reduced. On the other hand, if the thickness exceeds 3 mm, the weight of the sheet itself becomes too large, which may cause a discomfort feeling upon application to the skin. If the gel sheet is not thick as a whole, the thickness can be increased in part thereof to about 6 mm.

[Additive]

According to the adhesive gel sheet for a living body of the present invention, the polymer gel comprising the gel sheet may suitably be added with various additives depending on the intended use of the gel sheet (i.e., depending on application to cosmetics, quasi drugs and the like).

As such additives, in addition to the medicinal ingredients for the purposes of cosmetic and facial treatment and skin treatment, considered are humectants, thickeners, flavoring agents, coloring agents, stabilizers, antioxidants, ultraviolet absorbers, tackifiers, pH adjusters, chelating agents, surfactants, antiseptic agents and antibacterial agents.

The medicinal ingredients are not particularly limited as long as they have been used in the fields of drugs, quasi drugs, cosmetics, hygienic good and sundries. The medicinal ingredients same as those contained in the solution existing on the surface of the polymer gel to be applied to the skin may be used.

The blending amount of the medicinal ingredient is not defined specifically because the amount of effective ingredient varies depending on the raw material thereof. In general, the amount is preferably 0.001 to 80 % by mass with respect to the total amount of the adhesive gel sheet for a living body, more preferably 0.05 to 10 % by mass.

The humectants include, for example, ethylene glycol, propylene glycol, butylene glycol, diethylene glycol, dipropylene glycol, glycerin, diglycerin, sorbitol, maltitol, trehalose, raffinose, xylitol, glucose, fructose, mannitol, hyaluronic acid and salts thereof, glycols such as polyethylene glycol and polyglycerin, polyalcohols and polysaccharides. These may be used solely or in combination of two or more kinds.

As the thickeners, preferable are those which give a certain degree of viscosity to a mixture solution in a relatively small amount. For example, water soluble polymers such as polyethylene oxide and hydroxylethyl cellulose may be used. The blending amount of the thickener is preferably 0.01 to 20 % by mass with respect to the mixture solution comprising the polymer gel and/or the curing agent, more preferably 0.05 to 10 % by mass.

In the case of the gel which exudes the solution containing the medicinal ingredient to the surface upon application to the skin, it is effective to add inorganic electrolytes such as sodium chloride and potassium chloride to exude the solution more easily.

[Example of the process for producing the adhesive gel sheet for a living body]

Next, a process for producing the adhesive gel sheet according to the present invention will be explained specifically by way of an example, but the present invention is not limited thereto.

As the process for producing the polymer gel, there is a process of dissolving the above-described polymer compound in water or a solvent containing water and adding thereto a crosslinking factor, which is at least one kind of compound having a polycationic compound, to cause a crosslinking reaction. In general, the crosslinking reaction occurs immediately after the addition of the crosslinking factor. However, the rate of the crosslinking reaction may be adjusted by heating, cooling or pH adjustment, as required.

Here, mixing of the acidic polymer compound and the polycationic compound, in which the reaction time is short, is preferably performed using a stirring apparatus capable of continuous stirring in a short time. In this case, the stirring apparatus capable of continuous stirring signifies a stirring apparatus in which the introduction of the object to be mixed, the mixing and the taking-out of the resulting mixture are continuously performed as described in the section [crosslinking reaction]. More specifically, a static mixer and a reactive extruder are signified. Among them, the static mixer is preferable because reduction in molecular weight due to shearing of the polymer hardly occurs.

Stirring time in the apparatus, i.e., retention time in the apparatus, is desirably about 5 to 300 seconds, more desirably about 10 to 150 seconds. If the retention time is shorter than the above-described range, the mixing is not performed homogeneously. Further, if the retention time is longer than the above-described range, a system of the polymer compound which reacts at high crosslinking reaction rate and the polycationic compound causes the gel to be solidified easily in the apparatus. Further, there is also a problem in that the size of the apparatus is increased as the retaining amount increases. The retention time in the stirring apparatus may be determined within the above-described range so that the mixing of the polymer compound and the polycationic compound is sufficiently carried out. In the case of the static mixer, the retention time and the mixing ability can be adjusted by a flow rate, an inner diameter of the mixer, increase or decrease in the element number and a configuration. In the case of the reactive extruder, the adjustment can be performed by the number of revolutions of a screw, a configuration and a feeding amount.

A stirring element in the static mixer is preferably detachable so that cleaning of the inside can be easily carried out. Upon introducing the polymer compound and the polycationic compound into the stirring apparatus, the polycationic compound preferably takes the form of a solution or a slurry because the formation of lumps is prevented and homogeneous mixing is achieved easily.

In the case of supplying the polycationic compound in the slurry form, a stirrer or the like is provided for a slurry supplying part to stir the slurry in order to avoid nonuniformity in concentration due to precipitation. At this time, a thickener may be added to the slurry to increase the viscosity thereof, thereby slowing the precipitation.

[Adjustment of thickness]

Adjustment of the thickness of the polymer gel comprising the adhesive gel sheet for a living body according to the present invention is performed, for example, by spreading the gel-forming composite which is not solidified yet using an extruder, a doctor blade or a roller, or filling the gel-forming composite into a vessel having a predetermined thickness.

In the case of spreading the gel-forming composite into the sheet form using the blade or the roller, the blade or the roller may directly contact the composite solution. However, it is preferred to spread the composite solution in the state of being sandwiched between two film-shaped separators from the viewpoint of smoother finish of the gel surface and hygiene.

In the latter process of filling the gel-forming composite into the vessel having a predetermined thickness, the vessel may be shaped to fit with the shape of the adhesive gel sheet for a living body. This facilitates the total producing process up to the shaping of the gel, which is preferable in view of the producing process. Further, as compared with the process of forming a long belt-shaped gel and stamping it into the shape for intended use, this process is more suitable because it reduces the amount of wasted gel and hygienically eliminates contact between the gel and the stamping blade.

Further, in order to obtain the above-described polymer gel by the crosslinking reaction, the crosslinking factor may be added or heating may be performed to the composite before the crosslinking reaction. The timing of these operations may suitably be selected depending on the reaction systems. For example, in a system where the crosslinking reaction starts immediately after the addition of the crosslinking factor, the addition thereof and the mixing for homogenizing the composite are preferably performed immediately before the thickness adjustment or the filling in the above-described process.

Regarding the polymer gel of the present invention, a time for completing the solidification of the gel by the crosslinking reaction after the adjustment of the thickness varies from a short time of about 1 minute to several hours or several days. During the period until the solidification is finished, it is not preferable to apply to the gel a force which may transform the gel such as a locally applied pressure or a laterally deviating force. However, if the gel which does not yet complete the solidification is subjected to cutting, for example, the gel may be pressed in advance to give a cutting line and push the unsolidified composite away from the cutting line, thereby preventing the unsolidified liquid composite from spilling out of the cut section.

[Introduction of the base]

As a process of introducing a base material such as woven or nonwoven fabric having a large open area ratio in the gel, as described above, it is preferred to immerse the woven or nonwoven fabric in the gel-forming composite in the liquid state which does not yet complete the solidification and shape them into the sheet to solidify the gel. More specifically, in the case of forming the sheet by inserting the liquid gel-forming composite in a gap between two film-shaped separators arranged at a predetermined interval, woven or nonwoven fabric having a sufficiently large open area ratio is immersed in the gel-forming composite in a direction parallel to the separators and the gel, which are integrally solidified by the crosslinking reaction. Thus, the woven or nonwoven fabric is preferably introduced.

The gel sheet including the woven or nonwoven fabric can also be obtained by a process of pouring the gel-forming composite on the woven or nonwoven fabric spread in advance and pressurizing the gel-forming composite directly or over the film-shaped separator adhered thereon by using the blade or the roller to a predetermined thickness. In this case, however, bubbles adhered to the woven or nonwoven fabric are apt to remain in the gel.

In the case of adjusting the thickness of the gel by pouring the gel-forming composite in a gap between two rollers arranged horizontally and fixed at a predetermined interval, the woven or nonwoven fabric is passed from the top of the roller together with the composite, thereby the fabric is introduced in the gel. Also in this case, it is preferred to pass the film-shaped separators in contact with the rollers to sandwich the composite and the woven or nonwoven fabric. Further, at this time, doctor blades are provided for the two rollers, respectively, so that composite layers of a predetermined thickness are formed on each of the rollers and supplied to the gap between the rollers in an equal amount. Thereby, the woven or nonwoven fabric to be supplied between the gap can be positioned almost at the center in a direction of the gel thickness. Still further, by supplying the woven or nonwoven fabric in close contact with one of the rollers or the film passing on the roller, the woven or nonwoven fabric is positioned at one of the surfaces of the gel. After the thickness thereof is adjusted by the roller, the gel is kept in a horizontal position and solidified so that the woven or nonwoven fabric takes the top surface of the gel. Thereby, bubbles remaining in the gel can be reduced.

[Curing treatment]

In the present invention, if the curing treatment is performed partially or entirely on at least one of the surfaces of the gel sheet comprised of the polymer gel, it may be performed after or before the solidification of the polymer gel is completed.

In the present invention, a state where "the solidification of the polymer gel is completed" indicates a state in which the composite which is not gelated yet has got shape retainability through the crosslinking reaction.

Among the above-described curing treatments, in the case of using the curing agent as described above, the curing agent may be applied on at least one of the surfaces of the solidified gel by coating using a coater, printer or a brush or by spraying using a spray.

Even in the case of applying the curing agent on the gel surface before completing the solidification, the curing agent may be applied in the same manner as the above. However, it is desired that the gel-forming composite before the treatment has a viscosity higher than a certain degree. The viscosity is preferably 10,000 mPa·s or higher if the thickness adjustment has been done. If the viscosity of the gel-forming composite is below 10,000 mPa·s upon the treatment using the curing agent, the gel-forming composite and the curing agent are mixed, which makes difficult to show difference in crosslinking density between the surface and the inside of the resulting gel sheet. As a result, the medicinal ingredient may not be exuded sufficiently from the inside of the gel.

In the case of applying the curing agent before completing the solidification of the gel, the difference in crosslinking density between the surface and the inside of the resulting gel sheet can be made by applying the curing agent before the thickness adjustment of the gel-forming composite. In this case, the curing agent is held in a sheet capable of holding the curing agent therein (hereinafter the sheet is referred to as a holding sheet). The holding sheet is used as the film-shaped separator described above to contact the gel-forming composite before completing the solidification. Then, the thickness of the gel-forming composite before completing the solidification is adjusted using a doctor blade or a squeegee. As a result, the curing agent held in the holding sheet is transferred to the gel-forming composite, which is kept stand until the solidification of the gel is completed. Thereby, the intended gel sheet is obtained in which the crosslinking density on the surface and in the inside is varied.

In the case of performing the curing treatment before the thickness adjustment as in the above-described producing process, the viscosity of the gel-forming composite is preferably 5,000 to 50,000 mPa·s. If the viscosity is below 5,000 mPa·s, the gel-forming composite and the curing agent are mixed, which makes difficult to show the difference in crosslinking density between the surface and the inside of the resulting gel sheet. As a result, the medical ingredient may not be exuded sufficiently from the inside of the gel. On the other hand, if the viscosity exceeds 50,000 mPa·s, the adjustment of the thickness of the gel-forming composite becomes difficult.

As the holding sheet, any sheet can be used as long as the curing agent is held therein. For example, used are a plastic sheet, a sponge sheet, paper, woven fabric and nonwoven fabric. Among them, since the holding sheet is peeled off before using the adhesive gel sheet for a living body, the plastic sheet which is peeled easily is preferable. The holding sheet may be colored or have a pattern or characters printed thereon.

Preferable material of the plastic sheet may be low density polyethylene, high density polyethylene, polypropylene and polyethylene terephthalate. Further, the curing agent may be added with a thickener so that the curing agent can be held homogeneously in the holding sheet.

[Package]

The adhesive gel sheet for a living body according to the present invention is preferably packaged hermetically using a packaging film excellent in vapor blocking property so as not to dry the medicinal ingredient solution on the gel surface while being stored until it is put into use. Vapor permeability of the packaging film is preferably not more than 2.0 g/(m$^2$· 24h), more preferably not more than 1.0 g/(m$^2$ · 24h) at 40°C and relative humidity of 90%RH. If the vapor permeability is higher than this, the medicinal ingredient solution and moisture in the gel are lost during storage, which impairs the characteristics of the gel sheet of the present invention.

With respect to the package configurations, adoptable are those using a known plastic film, i.e., pouch type packages such as a three-sided pillow package, a horizontal type three-sided seal package and a four-sided seal package, as well as a blister type package in which the gel sheet is put in a vapor blocking film molded in the cup form and a top portion of the cup is sealed with the vapor blocking film. In the case of using the blister type cup, the gel sheet obtained by stamping the solidified polymer gel into the shape appropriate for a body part to which the sheet is applied is placed in a concave portion of the cup and the top of the cup is sealed. Alternatively, as described in the above section [Adjustment of thickness], the gel-forming composite may be directly poured into the cup and solidified.

[Shape]

The shape of the adhesive gel sheet for a living body according to the present invention is not particularly limited. The sheet may be in the shape of an ellipse, a circle, a heart, a semi-circle, a semi-ellipse, a square, a rectangular, a trapezoid, a triangle, a shape suited to the body part or a combination of them. The shape may suitably be designed so that the gel sheet is most properly adhered to the body part. Further, a convex portion or a concave portion for alignment may be formed in the center or the periphery of the gel sheet, or alternatively, a cut or a cut-off portion may be provided depending on the configuration of the body part. Thereby, the handleability of the gel sheet improves.

[Application method]

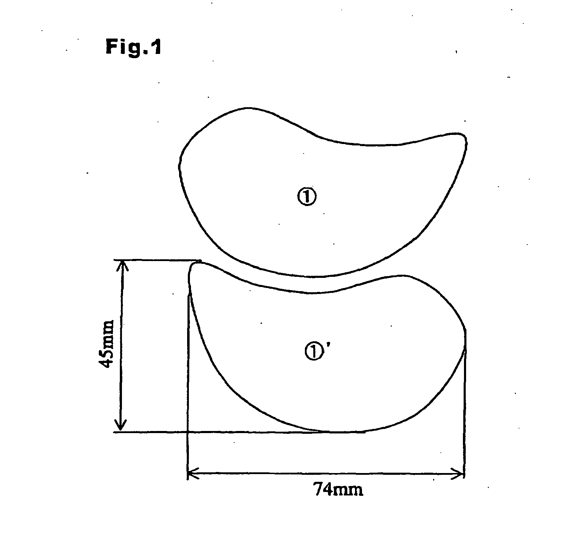

Body parts to which the adhesive gel sheet for a living body according to the present invention is applied may be a face (lips, cheeks, eye regions, regions above and beneath eyes, a nose, a forehead), a head, a chest, an abdomen, a back, a neck, arms, legs, fingers, nails and optic portions. The shape, area, thickness and adhesion property on the adhesive surface of the adhesive gel sheet for a living body may suitably be adjusted depending on the body part to which the sheet is applied. For example, in the case of forming a gel sheet to be applied over the whole face, the gel sheet may have the shape as shown in Fig. 1 including cut-off portions corresponding to the positions of the eyes and mouth and a cut corresponding to the position of the nose. Further, since the application area is large in this case, it is preferred to increase the adhesion ability of the adhesive surface or reduce the thickness.

From the hygienic viewpoint, the adhesive gel sheet for a living body according to the present invention preferably keeps the plastic film of polyethylene, polypropylene or polyethylene terephthalate adhered at least to the surface to be applied to the skin in the as-is state or in the state where the surface thereof is coated or baked with a releasing agent such as silicon resin so that it serves as a protection film until the sheet is actually put into use. The protection film may be cut to fit with the shape of the gel, or formed into the size larger than the gel so that more than one gel sheet is adhered thereto. The protection film may be colored or have a pattern or letters printed thereon.

Above all, it is preferred to adhere the gel sheets to be used at a time onto the protection film which is larger than a single gel sheet such that the surfaces to be applied to the skin contact the film and hermetically package the gel sheets and the protection film using the above-described packaging film. Thereby, a user can always take out only the necessary number of the gel sheets which are just-opened and succulent. In this case, any shape of the protection film (hereinafter referred to as a base film) is acceptable as long as the necessary number of the gel sheets can be adhered thereto in accordance with the shape thereof. More specifically, preferable is such a shape that allows the periphery of the gel to be arranged at 1mm or more inside the edge of the base film with the intention of preventing the medicinal ingredient solution on the gel surface from spilling out of the gel periphery and facilitating the peeling of the gel from the base film.

Material and thickness of the base film may suitably be selected depending on the thickness, weight and adhesive property of the gel sheet to be applied thereon. In the case of performing the step of stamping the gel into a predetermined shape on the base film, the base film is preferably made of a single film of oriented polyethylene terephthalate or oriented polypropylene or a laminate film of polyethylene or polypropylene and other resin because use of a relatively hard film allows better cutting of the gel.

The base film may have a transparent or translucent appearance. It may also be light-shielded, colored, pattern-printed or opaqued due to these treatments. In the case of giving the light-shielding effect on the gel sheet, an almost opaque film is adopted. Further, with the intention of having a design effect of emphasizing the transparency of the gel sheet or checking defects of the gel sheet during the production, a transparent or translucent film or a colored film which allows easy detection of the defects may be adopted.

The thickness of the base film is preferably 30 to 400 $\mu$m, more preferably 50 to 250 $\mu$m. If the film is thinner than this, cutting of the gel on the film by half-stamping becomes difficult and handling of the gel sheet taken out of the hermetical package becomes difficult due to lack of rigidity of the film. Further, if the film is thicker than this, the rigidity is higher than necessary, which is disadvantageous to the costs.

In order to prevent the drying of the gel sheet and from the hygienic aspect, a protective film (hereinafter referred to as a top film) is preferably adhered to the gel surface which is not applied to the skin. Material and thickness of the top film may suitably be selected depending on the thickness, weight and adhesive property of the gel sheet to be applied thereon. If the top film is stamped simultaneously in the step of stamping the gel into a predetermined shape, the top film is preferably made of a polyethylene film, a non-oriented polypropylene film, paper, nonwoven fabric or a composite thereof because a relatively soft material allows stamping without squashing of the gel.

The top film may have a transparent or translucent appearance. It may also be light-shielded, colored, pattern-printed or opaqued due to these treatments. In the case of giving the light-shielding effect on the gel sheet, an almost opaque film is adopted. Further, with the intention of having a design effect of emphasizing the transparency of the gel sheet or checking defects of the gel sheet during the production, a transparent or translucent film or a colored film which allows easy detection of the defects may be adopted.

The thickness of the top film is preferably 10 to 200 $\mu$m, more preferably 15 to 100 $\mu$m. If the film is thinner than this, the strength of the film itself becomes insufficient, which makes the handling of the film during the production difficult. If the film is thicker than this, the gel cannot be cut properly in the cutting step.

In the case of adhering films on both surfaces of the gel sheet, it is preferred to distinguish the films by shape, thickness, material, coloring and printing to prevent use of the gel sheet in a reversed state. With respect to the material, if a transparent hard film such as an oriented polyethylene terephthalate film or an oriented polyethylene film is used as the base film, the top film on the reverse side is preferably made of a soft film such as a polyethylene film or a non-oriented polypropylene film. Further, a high density polyethylene film having a slightly fogged mat appearance or a polyethylene film which is mat-finished by a chemical blast method or a sand blast method is more preferably used because it is easily distinguished from the base film. The distinction between the front and rear surfaces of the gel sheet by printing on the films is advantageous in that an explanation can be given by text. It is preferred to take measures on the printed surface to prevent deletion of the indication by the solvent in the gel, such as coating with a resin which does not elute in the solvent in the gel sheet. If the top film is made of a laminate film as described above in connection with the base film, the printing is preferably performed on the laminate surface. Similarly, in the case where the distinction is made by coloring the film, it is desired to use a pigmented colorant or provide a laminate or a coating as described above so that the colorant in the film does not transfer to the gel sheet.

The protective film adhered to the gel sheet is poorer in elasticity as compared with the gel sheet and hence cannot sufficiently follow the movement of the skin surface. Therefore, it is preferably peeled off upon use. However, until the gel sheet is adhered to an intended body part, the protective film is kept adhered to the gel surface which is not adhered to the skin, and then it is removed after the sheet is positioned to contact closely with the skin. Thereby, the gel sheet which is soft and difficult to handle by itself can easily be adhered to the predetermined position. The protective film may be a film served as the separator during the gel producing process or that separately adhered to the gel after the gel sheet production is finished.

Examples

Hereinafter, the present invention will be explained in further detail by way of examples and comparative examples. However, the present invention is not limited thereto.

[Production of adhesive gel sheet for a living body]

Example 1 (Whitening cosmetic pack for eye regions)

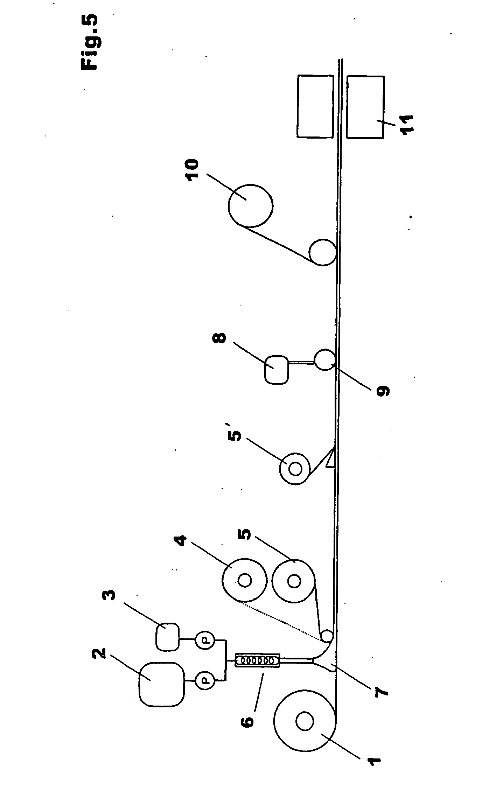

An adhesive gel sheet for a living body was made by the following producing process. Fig. 5 shows a structure of a producing apparatus used for making the adhesive gel sheet for a living body. A composition of the gel-forming composite and that of the curing agent are shown in Formula 1 in Table 1.

Ingredients of a polymer solution of Formula 1 were kneaded homogeneously in a stirring bath provided with an anchor rotor for about 30 minutes and kept stand for 10 hours to remove air bubbles. Further, ingredients of a polycationic compound solution were mixed with purified water to prepare slurry. The polymer solution and the slurry were fed in supply tanks (2 and 3) of two quantitative supply pumps connected to a static mixer (6) which is manufactured by Noritake Co., Ltd. and has an internal diameter of 17.5 cm and 96 elements. Flow rates of the supply pumps were adjusted so that the former took 75 and the latter took 25 in the weight ratio and both were mixed in the static mixer (6). As measured by a B-type viscometer, the viscosity of the polymer solution was about 12,000 mPa·s and that of the slurry was about 50 mPa · s. A gel-forming composite discharged from the mixer showed a viscosity of about 22,000 mPa·s and a pH of 5.0. Time required for passing the solution through the mixer was about 30 seconds.

The thus obtained gel-forming composite (7) was coated on a polypropylene sheet 1 of 100 $\mu$m thick, on which woven fabric (4) which is made of 15 denier nylon (6 nylon) monofilament (tricot half, N=0.44) and has a Base weight per m$^2$ of 17 g/m$^2$ and a biaxially-oriented polypropylene film (5) of 40 $\mu$m thick were covered in this order. They were spread uniformly to a thickness of 0.8 mm by using a roller thereon. Crosslinking reaction in the gel-forming composite proceeded during transfer on a belt conveyer. After 1 minute had elapsed since the coating, the polypropylene film at the top was peeled and wound up. Reference numeral 5' in the figure indicates a winding roller. At that time, the gel-forming composite did not adhere to a finger even if it was pressed against the gel surface and the woven fabric was almost buried in the gel.

Further, at the rearward position of the line, a curing agent in a tank (8) as indicated in Formula 1 of Table 1, i.e., a crosslinking factor (aluminum chloride hexahydrate) dissolved in a solvent (purified water), was uniformly coated on the gel surface using a coating roller (9), thereby the synthetic polymer gel was subjected to curing treatment. The coating amount of the curing agent was adjusted to 3 mg per cm$^2$ of the surface of the synthetic polymer gel (i.e., 1 $\times$ 10$^{-6}$ equivalent/cm$^2$ by crosslinking equivalent).

Further, a polypropylene sheet (10) of 50 $\mu$m thick was adhered to the surface of the synthetic polymer gel to which the curing agent had been coated and the resulting product was stamped using a cutter (11) into the shape shown in Fig. 1 (a pack for eye regions, ① for the right and ①' for the left; length $\times$ = 45 mm, width y = 74 mm). The gel sheet thus stamped was sealed into a four-sided sealing pouch which is made of a four-layered aluminum laminate film (structure: a 12 $\mu$m PET film/ a 9 $\mu$m aluminum foil/ a 12 $\mu$m PET film/ a 40 $\mu$m L-LDPE heat seal layer) and has vapor permeability of 0.01 g/(m$^2$·24h) or lower under the condition of 40°C and relative humidity of 90%RH. This was kept at room temperature for 3 days to obtain an intended final product.

Example 2 (Whitening cosmetic pack for whole face)

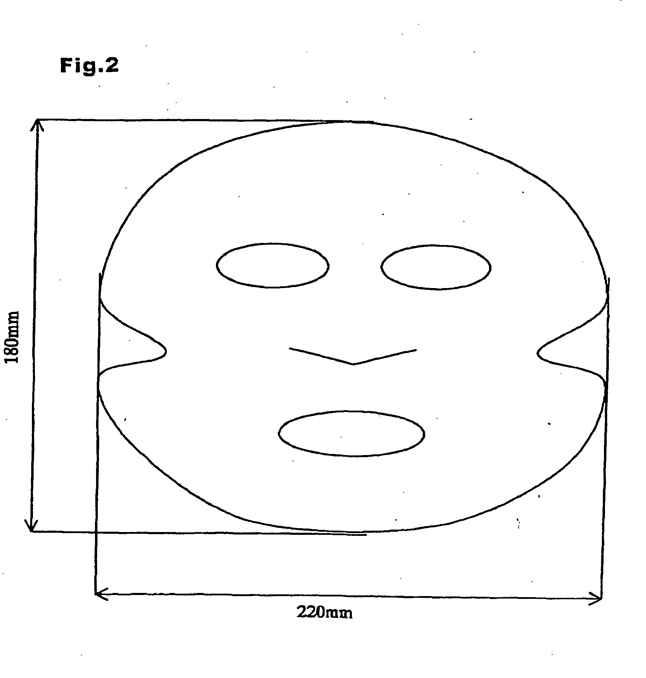

According to Formula 1 of Table 1, a gel sheet was formed and treated in the same manner as Example 1 except that the thickness of the gel was set to 1.0 mm. This was stamped into the shape shown in Fig. 2. The stamped gel was sealed into the four-sided sealing pouch and kept at room temperature for 3 days in the same manner as Example 1 to obtain an intended final product.

Example 3 (Whitening cosmetic pack for whole face)

A final product in the shape shown in Fig. 2 sealed in the same four-sided sealing pouch as that of Example 1 was obtained in the same manner as Example 2 except that Formula 2 of Table 1 was used. At that time, the viscosity of the polymer solution was about 12,200 mPa · s and that of the slurry was about 50 mPa·s. The gel-forming composite discharged from the mixer showed a viscosity of about 22,500 mPa·s and a pH of 5.0. Time required for passing the solution through the mixer was about 30 seconds.

Example 4 (Anti-aging pack for whole face)

A final product in the shape shown in Fig. 2 sealed in the same four-sided sealing pouch as that of Example 1 was obtained in the same manner as Example 2 except that Formula 3 of Table 1 was used. At that time, the viscosity of the polymer solution was about 12,500 mPa·s and that of the slurry was about 50 mPa·s. The gel-forming composite discharged from the mixer showed a viscosity of about 23,000 mPa·s and a pH of 5.1. Time required for passing the solution through the mixer was about 30 seconds.

Example 5 (skin-beautifying cosmetic pack for whole face)

A final product in the shape shown in Fig. 2 sealed in the same four-sided sealing pouch as that of Example 1 was obtained in the same manner as Example 2 except that Formula 4 of Table 1 was used. At that time, the viscosity of the polymer solution was about 12,200 mPa·s and that of the slurry was about 50 mPa·s. The gel-forming composite discharged from the mixer showed a viscosity of about 22,500 mPa·s and a pH of 5.0. Time required for passing the solution through the mixer was about 30 seconds.

Example 6 (Acne cosmetics)

A gel sheet of 0.8 mm thick was prepared in the same manner as Example 1 except that Formula 5 of Table 1 was used and the 15 denier nylon fabric was not used. The gel sheet was stamped into a round shape having a diameter of 20 mm and sealed into the same four-sided sealing pouch as that of Example 1 made of an aluminum laminate film, thereby obtaining a final product. At that time, the viscosity of the polymer solution was about 12,500 mPa·s and that of the slurry was about 50 mPa·s. The gel-forming composite discharged from the mixer showed a viscosity of about 23,000 mPa·s and a pH of 5.2. Time required for passing the solution through the mixer was about 30 seconds.

Example 7 (Liquid addition type whitening cosmetic pack for whole face)

A gel-forming composite obtained by using Formula 6 of Table 1 and the same static mixer as that of Example 1 was shaped into a sheet of 1.0 mm thick using a roller in the same manner as Example 1. At that time, the viscosity of the polymer solution was about 10,500 mPa·s and that of the slurry was about 50 mPa·s. The gel-forming composite discharged from the mixer showed a viscosity of about 19,000 mPa·s and a pH of 5.5. Time required for passing the solution through the mixer was about 30 seconds.

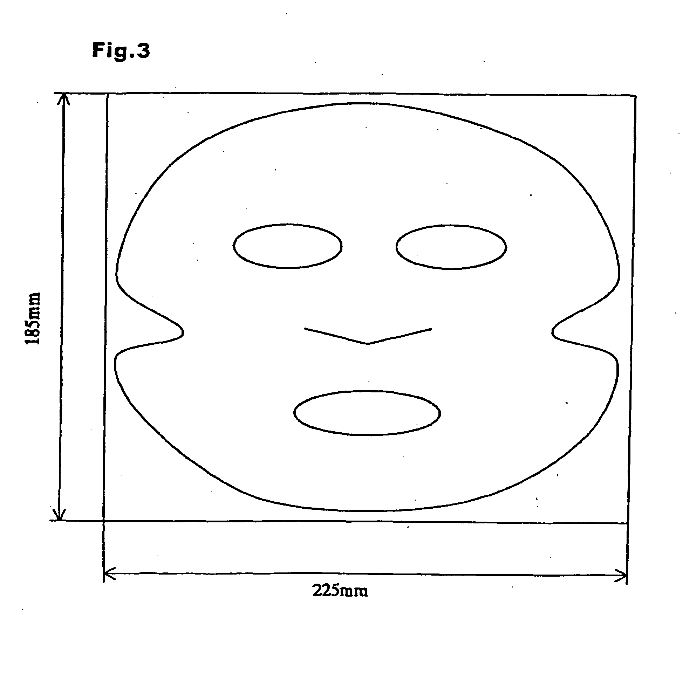

Subsequently, among the steps using the apparatus shown in Fig. 5, the peeling of the film with the winding roller (5'), the curing treatment through the coating of the curing agent contained in the tank (8) using the coating roller (9) and the adhesion of the film (10) were not carried out. Further, only the polypropylene sheet of 50 $\mu$m thick and the gel were cut using the cutter (11) from the 50 $\mu$m thick polypropylene sheet side so that the polypropylene sheet of 100 $\mu$m thick remained uncut. The gel and the 50 $\mu$m thick polypropylene sheet in an area surrounding the mask and an area corresponding to the eyes and nose, which were unnecessary, were removed. Then, the 100 $\mu$m thick polypropylene sheet was cut into the shape of a rectangular having length $\times$ = 185 mm and width y = 225 mm so that the gel (face mask) stayed almost at the center thereof, thereby obtaining the polymer gel in the shape shown in Fig.3.

On the polypropylene sheet on the periphery of the polymer gel, 2 g of a medicinal ingredient solution having a composition indicated in Formula 6 of Table 1 was dropped almost uniformly so that the solution came into contact with the cut section of the gel. The polymer gel was sealed in the same four-sided sealing pouch as that of Example 1 together with the polypropylene sheet bearing the medicinal ingredient solution and kept at room temperature for 3 days. Thus, an intended final product was obtained.

Example 8 (Whitening cosmetic pack for whole face)

A gel formed using Formula 1 of Table 1 was subjected to the curing treatment and stamped into the shape of a face mask in the same manner as Example 2 except that woven fabric of 30 denier nylon (6 nylon) multifilament yarn (N = 0.91) having the Base weight per m$^2$ of 50 g/m$^2$ was used instead of the woven fabric (tricot half) having the Base weight per m$^2$ of 17 g/m$^2$. The gel was hermetically packaged in the four-sided sealing pouch of an aluminum laminate film and kept at room temperature for 3 days to obtain an intended final product.

Example 9 (Whitening cosmetic pack for whole face)

A gel formed using Formula 1 of Table 1 was subjected to the curing treatment and stamped into the shape of a face mask in the same manner as Example 2 except that polyester nonwoven fabric having an average fiber amount of 10 denier and the Base weight per m$^2$ of 30 g/m$^2$ (N = 0.17) was used instead of the woven fabric (tricot half) having the Base weight per m$^2$ of 17 g/m$^2$. The gel was hermetically packaged in the four-sided sealing pouch of an aluminum laminate film and kept at room temperature for 3 days to obtain an intended final product.

Example 10 (Whitening cosmetic pack for eye regions contained in a vessel)

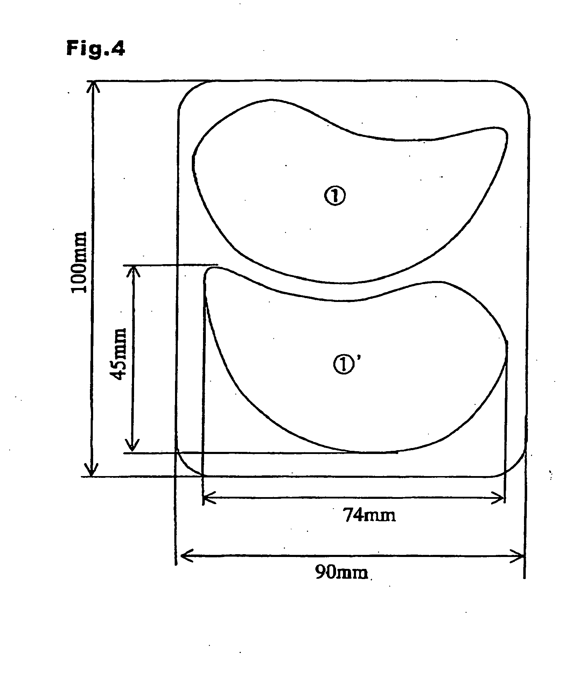

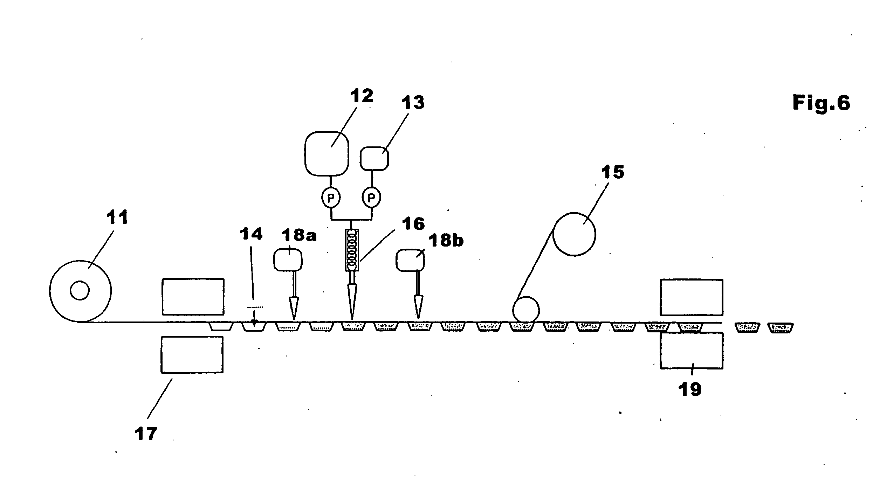

Fig. 6 shows a structure of a producing apparatus. A cup textile (11) of a vinyl chloride sheet (200 $\mu$m thick, vapor permeability under the condition of 40°C and relative humidity 90%RH was 0.1 g/ (m$^2$·24h)) was heat-molded into the shape of a dish having concave portions of 1.2 mm in depth (①, ①') as shown in Fig. 4. Reference numeral 17 in the figure indicates a heat molding apparatus. Woven fabric (14) (tricot half) of 15 denier nylon monofilament having the

Base weight per m$^2$ of 17 g/m$^2$, which was the same as that used in Example 2 and stamped into the shape identical with the concave portions ① and ①', was placed in the concave portions, respectively. Then, 3 g of a gel-forming composite obtained by homogeneously kneading ingredients of Formula 6 of Table 1 in the static mixer (16) in the same manner as Example 7 was poured into the concave portions, respectively. At that time, the viscosity of the polymer solution was about 10,500 mPa·s and that of the slurry was about 50 mPa·s. The gel-forming composite discharged from the mixer showed a viscosity of about 19,000 mPa·s and a pH of 5.5. Time required for passing the solution through the mixer was about 30 seconds.

Further, 0.3 g of a medicinal ingredient solution of Formula 6 of Table 1 was poured from a liquid dispenser (18b) onto the gel bodies in the concave portions, respectively, on which a cover film made of an easy peel film of four-layered aluminum laminate (structure: a 12 μm PET film/ a 9 μm aluminum foil/ a 12 μm PET film/ a 40 μm easy peel sealant layer) was heat-sealed to provide a hermetic package. The periphery of the dishes was stamped off using a cutter (19). This was kept at room temperature for 3 days to obtain an intended final product. In Fig. 6, reference numerals 12 and 13 indicate tanks for supplying the polymer solution and the polycationic slurry. Reference numeral 18a indicates a liquid dispenser, which was not used in this example.

Example 11 (Whitening cosmetic pack for eye regions contained in a vessel)

In the same manner as Example 10, a vinyl chloride sheet of 200 μm thick was heat-molded into the dish shape having concave portions of 1.2 mm in depth using the producing apparatus shown in Fig. 6. Woven fabric (14) (tricot half) of 15 denier nylon having the Base weight per m$^2$ of 17 g/m$^2$, which was stamped into the shape identical with ① and ①', was placed in the concave portions, respectively, on which a curing agent of Formula 7 of Table 1 was uniformly sprayed from the liquid dispenser 18a in an amount of about 0.1 g per concave portion. The spraying amount of the curing agent was about 4 mg per cm$^2$ of the surface of the synthetic polymer gel (i.e., $2.7 \times 10^{-6}$ equivalent/cm$^2$ in crosslinking equivalent). Further, 3 g of a gel-forming composite obtained by homogeneously kneading a polymer solution and a polycationic compound solution of Formula 7 of Table 1 in the static mixer in the same manner as Example 10 was poured into the concave portions, respectively. At that time, the viscosity of the polymer solution was about 12,000 mPa·s and that of the slurry was about 50 mPa·s. The gel-forming composite discharged from the mixer showed a viscosity of about 21,000 mPa·s and a pH of 5.5. Time required for passing the solution through the mixer was about 30 seconds.

Then, in the same manner as Example 10, a cover film made of an easy peel film of four-layered aluminum laminate was heat-sealed thereon to provide a hermetic package and the periphery of the concave portions was stamped off using the cutter. This was kept at room temperature for 3 days to obtain an intended final product.

Example 12 (Whitening cosmetic pack for eye regions)

Among ingredients indicated in Formula 8 of Table 1, those of a polymer solution were kneaded homogeneously for about 30 minutes and kept stand for 10 hours to remove air bubbles. At that time, the viscosity of the polymer solution was about 10,500 mPa·s and the pH was 8.2. Further, ingredients of an organic acid solution of Formula 8 of Table 1 were kneaded to prepare the organic acid solution. The viscosity of the organic acid solution was 10 mPa·s. The gel-forming composite, which was obtained by homogeneously kneading in the static mixer in the same manner as Example 7 except that the polycationic compound solution was replaced with the organic acid solution, was stacked with a tricot half and a polypropylene film of 50 μm thick and spread to a thickness of 0.8 mm thick by using a roller. Thereby, a polymer gel was obtained. The viscosity measured immediately after the mixing of the polymer solution and the organic acid solution was about 9,800 mPa·s and the pH was 5.7.

From the 50 μm thick polypropylene film side of the polymer gel, the polymer gel was stamped into the shape shown in Fig. 1 while leaving the polypropylene sheet of 100 μm thick uncut. The gel and the 50 μm thick polypropylene sheet on the peripheral area, which were unnecessary, were removed. Then, the 100 μm thick polypropylene film around the gel was cut into the shape of a rectangular having length x = 100 mm, width y = 90 and corners rounded by R = 10 mm so that the gel (a pack for eye regions) stayed almost at the center thereof. Thus, the polymer gel was obtained.

The resulting gel was sealed into the same four-sided sealing pouch as that used in Example 1 made of an aluminum laminate film and kept at room temperature for 3 days to obtain an intended final product.

Comparative Example 1

Ingredients indicated in Formula 1 of Table 1 were used in the same manner as Example 1. Among the ingredients, those of a polymer solution were kneaded in a batch type mixing apparatus having an anchor-shaped rotor for 30 minutes. Then, a polycationic compound solution prepared in another vessel was weighed to take 25 in the weight ratio with respect to 75 of the polymer solution, which was added to the mixing apparatus containing the polymer solution.

A crosslinking reaction proceeded as the mixing was continued. Then, after the lapse of 5 minutes, the mixing was not able to continue and hence the mixing apparatus was turned off and the content was taken out. The content had already been a shape retaining gel, which was not able to be spread into the sheet form.

Comparative Example 2

Ingredients indicated in Formula 1 of Table 1 were used in the same manner as Comparative Example 1. A polymer solution and a polycationic compound solution were mixed in a stirring mixer provided with an anchor rotor. After the lapse of 1 minute, the mixing was stopped and the gel-forming composite was taken out of the bottom of the vessel. Then, in the same manner as Example 1, the gel-forming composite was coated onto a polypropylene sheet of 100 $\mu$m thick, on which woven fabric (tricot half) of nylon having the Base weight per m$^2$ of 17 g/m$^2$ and a biaxially-oriented polypropylene film of 40 $\mu$m thick were covered in this order. They were spread uniformly to a thickness of 0.8 mm by using a roller. After the coating of the curing agent and the replacement of the film, the sheet was stamped into the shape shown in Fig. 1, which was sealed into a four-sided sealing pouch made of an aluminum laminate film and kept at room temperature for 3 days in the same manner as Example 1. Thus, an intended final product was obtained.

Comparative Example 3

In the same manner as Comparative Example 2 except that ingredients of Formula 8 of Table 1 were used, a polymer solution and an organic acid solution were prepared and mixed in the stirring mixer provided with the anchor rotor for 1 minute, which was then spread into the sheet form of 0.8 mm thick while including therein woven fabric (tricot half) of 15 denier nylon having the Base weight per m$^2$ of 17 g/m$^2$. The polymer solution showed a pH of 8.3 by itself and the gel-forming composite obtained after mixing with the organic acid solution showed a pH of 5.7.

In that state, it was stamped into the shape shown in Fig. 1, which was sealed into the four-sided sealing pouch made of an aluminum laminate film and kept at room temperature for 3 days. Thus, an intended final product was obtained.

With respect to the products obtained by the above-described examples and comparative examples, liquid existing on the surface to be applied to the skin was quantified by the following process. More specifically, a paper wiper manufactured by CRECIA Corporation (Kimwipe wiper S-200) was weighed sheet by sheet using a precision balance, the whole gel surface to be measured is wiped with it and the used wiper is weighed again using the precision balance. A difference between two weight values was defined as the liquid weight on the gel sheet surface. Further, liquid on the surface was collected to analyze whether or not the medicinal ingredient added was contained therein using a high performance liquid chromatograph.

| | Gel surface area | Liquid amount on surface | Liquid amount per surface area | Medicinal ingredient in liquid |
|---|---|---|---|---|
| Ex. 1 | 25.3 cm$^2$ | 0.03 g | 1.2 mg/cm$^2$ | present |
| Ex. 2 | 292 cm$^2$ | 0.34 g | 1.1 mg/cm$^2$ | present |
| Ex. 3 | 292 cm$^2$ | 0.33 g | 1.1 mg/cm$^2$ | present |
| Ex. 4 | 292 cm$^2$ | 0.30 g | 1.0 mg/cm$^2$ | present |
| Ex. 5 | 292 cm$^2$ | 0.33 g | 1.1 mg/cm$^2$ | present |
| Ex. 6 | 3.1 cm$^2$ | 0.003 g* | 1.0 mg/cm$^2$ | present |
| Ex. 7 | 292 cm$^2$ | 0.56 g | 1.9 mg/cm$^2$ | present |
| Ex. 8 | 292 cm$^2$ | 0.29 g | 1.0 mg/cm$^2$ | present |
| Ex. 9 | 292 cm$^2$ | 0.31 g | 1.1 mg/cm$^2$ | present |
| Ex. 10 | 25.3 cm$^2$ | 0.11 g | 4.3 mg/cm$^2$ | present |
| Ex. 11 | 25.3 cm$^2$ | 0.02 g | 0.8 mg/cm$^2$ | present |
| Ex. 12 | 25.3 cm$^2$ | 0.03 g | 1.2 mg/cm$^2$ | present |
| Com. Ex. 1 | - | - | - | - |
| Com. Ex. 2 | 25.3 cm$^2$ | 0.02 g | 0.8 mg/cm$^2$ | present |
| Com. Ex. 3 | 25.3 cm$^2$ | 0.01 g | 0.4 mg/cm$^2$ | present |

*Average value obtained by measuring the liquid amount on 10 sheets at one time.

Ten expert panelists used each of the products obtained above for 2 weeks by applying the product on the face for 20 minutes once a day to make evaluations on each evaluation item according to the criteria of the following table. The total score of all the panelists was recognized as the evaluation results. Accordingly, a greater score indicates a higher advantage on the evaluation item. Further, transparency of the gel sheet was evaluated on the criteria of "transparent" in which black characters of 10 points written on a white background can be read easily through the gel sheet placed on the characters, "translucent" in which the characters are hard to see but readable and "opaque" in which the characters are completely illegible. The results are shown below.

| Criteria | Score |
|---|---|
| Highly effective | 5 |
| Effective | 4 |
| Rather effective | 3 |
| Little effective | 2 |
| Not effective | 1 |

<Effect>

| | Moisturizing feeling | Duration of cooling feeling | Adhesion feeling | Medicinal Effect* | Ease of handling | Transparency |
|---|---|---|---|---|---|---|
| Ex. 1 | 45 | 43 | 46 | 40 | 15 | transparent |
| Ex. 2 | 42 | 42 | 41 | 41 | 35 | transparent |
| Ex. 3 | 46 | 45 | 45 | 37 | 32 | transparent |
| Ex. 4 | 45 | 44 | 43 | 38 | 33 | transparent |
| Ex. 5 | 43 | 43 | 45 | 42 | 35 | transparent |
| Ex. 7 | 45 | 43 | 43 | 43 | 35 | transparent |
| Ex. 8 | 43 | 41 | 37 | 43 | 39 | translucent |
| Ex. 9 | 41 | 43 | 35 | 41 | 41 | transparent |
| Ex. 10 | 41 | 45 | 47 | 35 | 41 | transparent |
| Ex. 11 | 34 | 42 | 44 | 35 | 39 | transparent |
| Ex. 12 | 31 | 41 | 41 | 37 | 43 | transparent |
| Com. Ex. 2 | 13 | 40 | 12 | 28 | 11 | transparent |
| Com. Ex. 3 | 13 | 35 | 13 | 28 | 12 | transparent |

Medicinal effect: Whitening effect in Examples 1, 2, 3, 7, 8, 9, 10, 11, 12 and Comparative Examples, anti-aging effect in Example 4, skin-beautifying effect in Example 5

The reason why the evaluations on the adhesion feeling and the ease of handling were low in Comparative Examples 2 and 3 was that the gel sheets were not cured sufficiently in parts, which remained on the face or fingers upon application or peeling.

Further, 10 panelists having pimples used the sample of Example 6 every day for a week and the following evaluation results were obtained.

| | Moisturizing feeling | Duration of cooling feeling | Adhesion feeling | Medicinal effect | Ease of handling | Transparency |
|---|---|---|---|---|---|---|
| Ex. 6 | 33 | 31 | 45 | 39 | 42 | transparent |

As described above, the adhesive gel sheet for a living body according to the present invention is excellent in moisturizing feeling derived from the medicinal ingredient solution existing on the surface thereof. The usability is also favorable due to the soft adhesion feeling, transparency, as well as fine homogeneity. Further, the effect of the added medicinal ingredient is sufficiently caused by the occlusive effect.

Table 1

| | | Formula 1 | Formula 2 | Formula 3 | Formula 4 | Formula 5 | Formula 6 | Formula 7 | Formula 8 |
|---|---|---|---|---|---|---|---|---|---|
| | | Exs. 1, 2, 8, 9, Com. Exs. 1, 2 | Ex. 3 | Ex. 4 | Ex. 5 | Ex. 6 | Exs. 7, 10 | Ex. 11 | Ex. 12, Com. Ex. 3 |
| Polymer solution | | | | | | | | | |
| Polymer | Polyacrylic acid (molecular weight 10,000 apx.) | 8.5 | 8.5 | 8.5 | 8.5 | 8.5 | 6.0 | 6.0 | - |
| | Sodium polyacrylate (molecular weight 4 million apx.) | 4.0 | 4.0 | 4.0 | 4.0 | 4.0 | 4.0 | 4.0 | 3.8 |
| Humectant | Dipropylene glycol | 10.0 | 10.0 | 10.0 | 10.0 | 10.0 | 10.0 | 3.0 | 7.0 |
| | Triethylene glycol | - | - | - | - | - | - | 7.0 | 3.0 |
| pH adjuster | Tartaric acid | - | - | - | - | - | 0.8 | 0.8 | - |
| | Citric acid | 0.2 | 0.2 | 0.2 | 0.2 | 0.2 | - | - | - |
| Polycation | Synthetic aluminum silicate (Al content: 5.6%) | - | - | - | - | - | - | - | - |
| Antiseptic agent | Sodium dehydroacetate | 0.3 | 0.3 | 0.3 | 0.3 | 0.3 | 0.3 | 0.3 | 0.3 |

(continued)

| | | Formula 1 | Formula 2 | Formula 3 | Formula 4 | Formula 5 | Formula 6 | Formula 7 | Formula 8 |
|---|---|---|---|---|---|---|---|---|---|
| | | Exs. 1, 2, 8, 9, Com. Exs. 1, 2 | Ex. 3 | Ex. 4 | Ex. 5 | Ex. 6 | Exs. 7, 10 | Ex. 11 | Ex. 12, Com. Ex. 3 |
| Medicinal ingredient | Vitamin C derivatives | 2.0 | - | - | - | - | - | 2.0 | 2.0 |
| | Kojic acid | - | 0.5 | - | - | - | - | 0.1 | 0.1 |
| | Arbutin | - | 3.0 | - | - | - | - | 1.0 | 1.0 |
| | Ellagic acid | - | 0.5 | - | - | - | - | - | - |
| | Rucinol | - | 0.3 | - | - | - | - | - | - |
| | Chamomile extract | - | 3.0 | - | - | - | - | - | - |
| | Vitamin E derivatives | - | - | 0.1 | - | - | - | - | - |
| | Dipotassium glycyrrhizinate | - | - | 0.1 | - | - | - | - | - |
| | Nicotinamide | - | - | - | 1.0 | - | - | - | - |
| | Pyridoxine hydrochloride | - | - | - | 0.001 | - | - | - | - |
| | Isopropylmethylphenol | - | - | - | - | 0.1 | - | - | - |
| | Zinc paraphenolsulfonate | - | - | - | - | 0.1 | - | - | - |
| Solvent | Purified water | 50.0 | 44.7 | 51.8 | 51.0 | 51.8 | 53.9 | 50.8 | 55.5 |
| Subtotal | | 75 | 75 | 75 | 75 | 75 | 75 | 75 | 75 |
| Polycationic compound suspension | | | | | | | | | |
| | Synthetic aluminum silicate (Al content: 5.6%) | 2.3 | 2.3 | 2.3 | 2.3 | 2.3 | 2.3 | 3.1 | - |
| Solvent | Purified water | 22.7 | 22.7 | 22.7 | 22.7 | 22.7 | 22.7 | 21.9 | - |
| Subtotal | | 25 | 25 | 25 | 25 | 25 | 25 | 25 | - |
| Organic acid solution - | | | | | | | | | |
| pH adjuster | Tartaric acid | - | - | - | - | - | - | - | 1.8 |

(continued)

| | | Formula 1 | Formula 2 | Formula 3 | Formula 4 | Formula 5 | Formula 6 | Formula 7 | Formula 8 |
|---|---|---|---|---|---|---|---|---|---|
| | | Exs. 1, 2, 8, 9, Com. Exs. 1, 2 | Ex. 3 | Ex. 4 | Ex. 5 | Ex. 6 | Exs. 7, 10 | Ex. 11 | Ex. 12, Com. Ex. 3 |
| Solvent | Purified water | - | - | - | - | - | - | - | 23.2 |
| Subtotal | | - | - | - | - | - | - | - | 25 |
| Curing agent | | | | | | | | | |
| Crosslinking factor | Aluminum chloride hexahydratc (Al content: 11%) | 10 | 10 | 10 | 10 | 10 | - | - | - |
| | Iron chloride (Fe content: 35%) | - | - | - | - | - | - | 10 | - |
| Solvent | Purified water | 90 | 90 | 90 | 90 | 90 | - | 90 | - |
| Medicinal ingredient solution | | | | | | | | | |
| Medicinal ingredient | Vitamin C derivatives | - | - | - | - | - | 5 | - | - |
| Humectant | Dipropylene glycol | - | - | - | - | - | 10 | - | - |
| Solvent | Purified water | - | - | - | - | - | 85 | - | - |

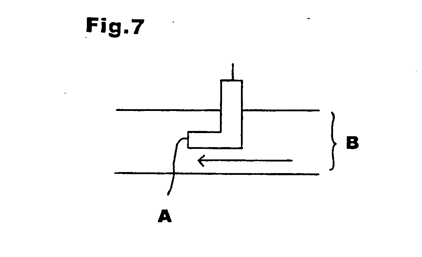

An upper limit of the viscosity of the solutions suitable for mixing in the static mixer is 200,000 mPa. If the viscosity exceeds the limit, pressure in the mixer needs to set extremely high for achieving the stirring effect, which remarkably impairs practicality. Further, the mixing ratio between the solutions to be stirred is preferably set such that the ratio of a solution in the smallest amount is 1 % by mass or higher with respect to the ratio of a solution in the largest amount. If there is a great difference in the mixing ratio, it is effective to arrange an opening (A) of a tube, through which the solution in the smaller addition amount is merged into the solution in the larger addition amount, at the center of a flow path (B) of the solution in the larger addition amount as shown Fig. 7. Thereby, the mixing is performed homogeneously.

Effect of the Invention

According to the present invention, provided are a process for producing an adhesive gel sheet for a living body which

(1) totally satisfies moisture retaining ability, cooling feeling, adhesion property and occlusive feeling to the skin,
(2) stays on the skin surface without peeling or falling off even if an excessive adhesion ability is not given thereto and causes the action of the medicinal ingredient to the skin more effectively,
(3) is easily applied and has transparency to such a degree that a visually uncomfortable feeling is not caused, and
(4) is produced by simple steps and the adhesive gel sheet obtained by the process.

Further, according to the present invention, also provided is a skin-care method using the above-described adhesive gel sheet for a living body, wherein various medicinal ingredients (skin-care ingredients) having ameliorating effects on a variety of skin troubles; such as roughness, dryness, somberness, rings under the eyes and freckles, are effectively worked in a simple manner and in a short time.

**Claims**

1. A process for producing an adhesive gel sheet for a living body, comprising mixing a synthetic polymer compound that dissolves in an amount of 1g or more in 100 g of water at 20°C, having an anionic functional group, with a polycationic compound capable of crosslinking the polymer compound in the presence of an aqueous medium to prepare a gel-forming composite having a pH of 3 to 7, impregnating the gel-forming composite into a woven or nonwoven fabric while the viscosity of the composite is 1,000 to 30,000 mPa·s shaping the composite-impregnated fabric into a sheet and crosslinking the composite in this state to obtain an adhesive gel sheet for a living body having a transparent or translucent polymer gel, the crosslinking thereof being completed in this state to obtain a polymergel solidified in one piece with the woven or nonwoven fabric, subjecting the adhesive gel sheet to curing treatment using a crosslinking agent on an optional part of at least one of the surfaces of the adhesive gel sheet wherein the woven or nonwoven fabric has a hypotherical shielding area N ($m^2/m^2$) by fibers per $m^2$ in the range of $0.05 \leq N \leq 2.0$ wherein N is represented by the following equation:

$$N = 0.107W \sqrt{\frac{1}{d\rho}}$$

wherein W is a Base weight per $m^2$ of the woven or nonwoven fabric represented by $g/m^2$, d is a denier amount of fibers comprising the fabric an $\rho$ is a density of a component of the fibers represented by $g/cm^3$, and $3 \leq W \leq 50$ $g/m^2$.

2. A process according to claim 1, wherein the polymer gel contains a medicinal ingredient, which is included in the polymer gel by addition to the gel-forming composite in advance, coating or dropping onto the inside of the mold in advance, or coating or dropping onto the sheet.

3. A process according to claim 1 or 2, wherein the preparation of the gel-forming composite and the shaping thereof into the sheet are performed sequentially.

**4.** A process according to any one of claims 1 to 3, wherein the medicinal ingredient is added to the polymer gel by coating or dropping the medicinal ingredient in a state of being dissolved or suspended in an acqueous medium onto a surface or a side of the sheet.

**5.** A process according to any one of claims 1 to 4, wherein, after the sheet is formed and the medicinal ingredient is contained therein, an optional part of at least one of the surfaces of the sheet is subjected to curing treatment using a crosslinking agent.

**6.** A process according to claim 5, wherein the crosslinking agent is a polycationic compound.

**7.** A process according to any one of claims 1 to 6, wherein the anionic functional group is a carboxyl group or a sulfonic acid group.

**8.** A process according to any one of claims 1 to 7, wherein the polycationic compound is a compound containing metal having a valence of 3 or more.

**9.** An adhesive gel sheet for a living body obtained by a process according to any one of claims 1 to 8, wherein a medicinal ingredient exists at least on a surface to be adhered to a living body.

**10.** A sheet according to claim 9 which is hermetically packaged by a packaging film having vapor permeability of 2,0 g/(m$^2$·24h) or lower at 40°C and relative humidity of 90% RH.

**11.** A sheet according to claim 9 or 10, wherein a plastic film is adhered to one or both of the surfaces of the adhesive gel sheet for a living body.

**12.** A sheet according to any one of claims 9 to 11, wherein a solution containing the medicinal ingredient is exuded to the sheet surface upon application to a living body.

**13.** An adhesive gel sheet according to any one of claims 9-12 for use in a skin-care method of a living body to provide partially or entirely a solution or a dispersion of the medicinal ingredient between a skin surface of the living body and the sheet, thereby taking care of a skin.

**Patentansprüche**

**1.** Ein Verfahren zur Herstellung einer haftenden Gelfolie für einen lebenden Körper, das Folgendes umfasst: das Mischen einer synthetischen Polymerverbindung, die sich in einer Menge von 1 g oder mehr in 100 g Wasser bei 20°C auflöst und eine anionische funktionelle Gruppe hat, mit einer polykationischen Verbindung, die zur Quervernetzung der Polymerverbindung in Anwesenheit eines wässerigen Mediums in der Lage ist, zur Herstellung eines Gel bildenden Verbundstoffs mit einem pH von 3 bis 7, das Imprägnieren des Gel bildenden Verbundstoffs zu einer gewebten oder nicht-gewebten Faser, während die Viskosität des Verbundstoffs 1.000 bis 30.000 mPa·s beträgt; das Formen des Verbundstoff-imprägnierten Stoffs zu einer Folie und die Quervernetzung des Verbundstoffs in diesem Zustand zur Herstellung einer haftenden Gelfolie für einen lebenden Körper, die ein transparentes oder lichtdurchlässiges Polymergel hat, wobei die Quervernetzung davon in diesem Zustand abgeschlossen wird, zur Gewinnung eines Polymergels, das mit der gewebten oder nicht-gewebten Faser zu einem Stück verfestigt ist, das Unterziehen der haftenden Gelfolie einer Härtungsbehandlung unter Verwendung eines Quervernetzungsmittels an einem fakultativen Teil von mindestens einer der Oberflächen der haftenden Gelfolie, worin die gewebte oder nicht-gewebte Faser einen hypothetischen Abschirmbereich N (m$^2$/m$^2$) mit Fasern pro m$^2$ im Bereich von $0,05 \leq N \leq 2,0$ hat, worin N durch folgende Gleichung dargestellt wird:

$$N = 0,107W \sqrt{\frac{1}{d\rho}}$$

worin W ein Basisgewicht pro m$^2$ der gewebten oder nicht-gewebten Faser, angegeben in g/m$^2$, ist, d eine Denier-Menge von Fasern ist, welche den Stoff umfassen, und $\rho$ eine Dichte einer Komponente der Fasern ist, die angegeben wird in g/cm$^3$, und $3 \leq W \leq 50$ g/m$^2$.

**2.** Ein Verfahren gemäß Anspruch 1, worin das Polymergel einen medizinischen Inhaltsstoff enthält, der in das Polymergel durch das Hinzufügen zum Gel bildenden Verbundstoff im Voraus, die Beschichtung oder Auftropfen auf die Innenseite der Form im Voraus oder die Beschichtung oder Auftropfen auf die Folie eingeschlossen wird.

**3.** Ein Verfahren gemäß Anspruch 1 oder 2, worin die Herstellung des Gel bildenden Verbundstoffs und seine Formung zur Folie nacheinander durchgeführt werden.

**4.** Ein Verfahren gemäß einem beliebigen der Ansprüche 1 bis 3, worin der medizinische Inhaltsstoff dem Polymergel hinzugefügt wird durch Beschichtung oder Auftropfen des medizinischen Inhaltsstoffs, in einem Zustand, in dem er in einem wässerigen Medium aufgelöst oder suspendiert ist, auf eine Oberfläche oder eine Seite der Folie.

**5.** Ein Verfahren gemäß einem beliebigen der Ansprüche 1 bis 4, worin, nachdem die Folie geformt wurde und der medizinische Inhaltsstoff darin enthalten ist, ein fakultativer Teil von mindestens einer der Oberflächen der Folie mit Hilfe eines Vernetzungsmittels einer Härtungsbehandlung unterzogen wird.

**6.** Ein Verfahren gemäß Anspruch 5, worin das Vernetzungsmittel eine polykationische Verbindung ist.

**7.** Ein Verfahren gemäß einem beliebigen der Ansprüche 1 bis 6, worin die anionische funktionelle Gruppe eine Carboxylgruppe oder eine Sulfonsäuregruppe ist.

**8.** Ein Verfahren gemäß einem beliebigen der Ansprüche 1 bis 7, worin die polykationische Verbindung eine Verbindung ist, die Metall mit einer Valenz von 3 oder mehr enthält.

**9.** Eine haftende Gelfolie für einen lebenden Körper, gewonnen durch ein Verfahren gemäß einem beliebigen der Ansprüche 1 bis 8, worin ein medizinischer Inhaltsstoff mindestens auf einer Oberfläche, die an einen lebenden Körper anzukleben ist, vorhanden ist.

**10.** Eine Folie gemäß Anspruch 9, die hermetisch mit einer Verpackungsfolie verpackt wird, welche eine Dampfdurchlässigkeit von 2,0 g/(m²·24h) oder weniger bei 40°C und eine relative Feuchtigkeit von 90% RH hat.

**11.** Eine Folie gemäß Anspruch 9 oder 10, worin ein Kunststofffilm auf eine oder beide der Oberflächen der haftenden Gelfolie für einen lebenden Körper angeklebt wird.

**12.** Eine Folie gemäß einem beliebigen der Ansprüche 9 bis 11, worin eine Lösung, welche den medizinischen Inhaltsstoff enthält, nach Auftragen auf einen lebenden Körper auf die Folienoberfläche abgesondert wird.

**13.** Eine haftende Gelfolie gemäß einem beliebigen der Ansprüche 9-12 zur Verwendung in einem Hautpflegeverfahren für einen lebenden Körper, um teilweise oder vollständig eine Lösung oder Dispersion des medizinischen Inhaltsstoffs zwischen einer Hautoberfläche des lebenden Körpers und der Folie bereitzustellen, wodurch die Haut gepflegt wird.

**Revendications**

**1.** Procédé de production d'une feuille de gel adhésive pour corps vivant, comprenant le mélange d'un composé polymère synthétique qui se dissout en une quantité de 1 g ou plus dans 100 g d'eau à 20 °C, contenant un groupe fonctionnel anionique, avec un composé polycationique capable de réticuler le composé polymère en présence d'un milieu aqueux pour préparer un composite formant un gel ayant un pH de 3 à 7, l'imprégnation du composite formant un gel dans un tissu tissé ou non tissé alors que la viscosité du composite est de 1 000 à 30 000 mPa·s, la mise sous forme de feuille du tissu imprégné de composite et la réticulation du composite à ce stade pour obtenir une feuille de gel adhésive pour corps vivant contenant un gel polymère transparent ou translucide, sa réticulation étant terminée à ce stade pour obtenir un gel polymère solidifié d'une seule pièce avec le tissu tissé ou non tissé, la soumission de la feuille de gel adhésive à un traitement de durcissement au moyen d'un agent de réticulation sur une partie facultative d'au moins une des surfaces de la feuille de gel adhésive, dans lequel le tissu tissé ou non tissé possède une surface de protection hypothétique $N$ (m²/m²) par fibre par m² dans la plage de $0,05 \leq N \leq 2,0$, où $N$ est représenté par l'équation suivante :

$$N = 0{,}107W \sqrt{\frac{1}{dp}}$$

dans laquelle W est un poids de base par m$^2$ du tissu tissé ou non tissé représenté en g/m$^2$, d est une quantité de denier de fibres constituant le tissu et p est une masse volumique d'un composant des fibres représenté en g/cm$^3$, et $3 \leq W \leq$ g/m$^2$.

2. Procédé selon la revendication 1, dans lequel le gel polymère contient un ingrédient médicinal qui est inclus dans le gel polymère par ajout au composite formant un gel à l'avance, enduction ou coulage à l'intérieur du moule à l'avance, ou enduction ou coulage sur la feuille.

3. Procédé selon la revendication 1 ou 2, dans lequel la préparation du composite formant un gel et sa mise sous forme de feuille sont réalisées séquentiellement.

4. Procédé selon l'une quelconque des revendications 1 à 3, dans lequel l'ingrédient médicinal est ajouté au gel polymère par enduction ou coulage de l'ingrédient médicinal se trouvant dans un état dissous ou suspendu dans un milieu aqueux sur une surface ou un côté de la feuille.

5. Procédé selon l'une quelconque des revendications 1 à 4, dans lequel, après la formation de la feuille et l'introduction de l'ingrédient médicinal dans cette dernière, une partie facultative d'au moins une des surfaces de la feuille est soumise à un traitement de durcissement au moyen d'un agent de réticulation.

6. Procédé selon la revendication 5, dans lequel l'agent de réticulation est un composé polycationique.

7. Procédé selon l'une quelconque des revendications 1 à 6, dans lequel le groupe fonctionnel anionique est un groupe carboxylique ou un groupe acide sulfonique.

8. Procédé selon l'une quelconque des revendications 1 à 7, dans lequel le composé polycationique est un composé contenant un métal ayant une valence de 3 ou plus.

9. Feuille de gel adhésive pour corps vivant, obtenue par un procédé selon l'une quelconque des revendications 1 à 8, dans laquelle un ingrédient médicinal existe au moins sur une surface à coller sur un corps vivant.

10. Feuille selon la revendication 9, qui est hermétiquement conditionnée par un film d'emballage présentant une perméabilité à la vapeur inférieure ou égale à 2,0 g/(m$^2$·24h) à 40 °C et à une humidité relative de 90 %.

11. Feuille selon la revendication 9 ou 10, dans laquelle un film plastique est collé sur une ou les deux surfaces de la feuille de gel adhésive pour corps vivant.

12. Feuille selon l'une quelconque des revendications 9 à 11, dans laquelle une solution contenant l'ingrédient médicinal est exsudée sur la surface de la feuille lors de son application sur un corps vivant.

13. Feuille de gel adhésive selon l'une quelconque des revendications 9 à 12, utilisable dans un procédé de soin cutané d'un corps vivant pour fournir partiellement ou entièrement une solution ou une dispersion de l'ingrédient médicinal entre une surface cutanée du corps vivant et la feuille, pour ainsi prendre soin d'une peau.

**Fig.1**

45mm

74mm

**Fig.2**

**Fig.3**

**Fig.4**

**Fig.5**

**Fig.6**

EP 1 327 442 B1

**Fig.7**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP SHO5449334 B
- JP SHO5592306 B
- JP HEI1254612 B
- JP 2000119129 A
- JP HEI386806 B